# EUROPÄISCHE PATENTSCHRIFT

(11) **EP 1 825 257 B1**
(45) Veröffentlichungstag und Bekanntmachung des Hinweises auf die Patenterteilung: **21.03.2012**
(21) Anmeldenummer: 05813728.2
(22) Anmeldetag: 24.11.2005
(51) Int. Cl.: G01N 33/487

(54) **DIAGNOSESYSTEM ZUM ERMITTELN VON STOFFKONZENTRATIONEN IN FLÜSSIGEN PROBEN**
DIAGNOSTIC SYSTEM FOR DETERMINING SUBSTANCE CONCENTRATIONS IN LIQUID SAMPLES
SYSTEME DE DIAGNOSTIC DESTINE A LA DETERMINATION DE CONCENTRATIONS DE SUBSTANCES DANS DES ECHANTILLONS LIQUIDES

(30) Priorität: 29.11.2004 DE 102004057503
(43) Veröffentlichungstag der Anmeldung: 29.08.2007
(73) Patentinhaber: F. Hoffmann-La Roche AG, 4070 Basel (CH); Roche Diagnostics GmbH, 68305 Mannheim (DE)
(72) Erfinder: BAINCZYK, Gregor, 68199 Mannheim (DE); MARQUANT, Michael, 68309 Mannheim (DE); WIEDER, Herbert, 68259 Mannheim (DE)
(74) Vertreter: Stößel, Matthias
(86) Internationale Anmeldenummer: PCT/EP2005/012568
(87) Internationale Veröffentlichungsnummer: WO 2006/058653

(56) Entgegenhaltungen:
- EP-A- 1 048 310
- WO-A-03/082091
- WO-A-2004/078233
- US-B1- 6 540 672

## Beschreibung

### Gebiet der Erfindung

Die Erfindung betrifft ein Diagnosesystem zum Ermitteln von Stoffkonzentrationen in flüssigen Proben, insbesondere zur Messung der Blutglukosekonzentration. Weiterhin betrifft die Erfindung ein Verfahren zum Bedienen eines erfindungsgemäßen Diagnosesystems.

### Stand der Technik

Die Überwachung der Blutglukosekonzentration ist für Diabetiker ein essentieller Bestandteil des Tagesablaufs. Dabei muss die Blutglukosekonzentration schnell, einfach mehrmals am Tag bestimmt werden, um gegebenenfalls entsprechende medizinische Maßnahmen ergreifen zu können. Um den Tagesablauf des Diabetikers nicht mehr als nötig einzuschränken, werden dabei häufig entsprechende mobile Geräte eingesetzt, welche Platz sparend und einfach zu handhaben sind, so dass die Blutglukosemessung beispielsweise am Arbeitsplatz oder auch in der Freizeit erfolgen kann.

Derzeit sind verschiedene mobile Geräte auf dem Markt, welche teilweise nach unterschiedlichen Messmethoden funktionieren. Dabei kommen verschiedene Diagnoseverfahren zum Einsatz, beispielsweise optische oder elektrochemische Messverfahren. Ein Beispiel eines häufig eingesetzten Messverfahrens nutzt eine spezielle Art elektrochemischer Teststreifen. Diese Teststreifen sind beispielsweise so aufgebaut, dass eine vorgegebene Blutmenge über ein Kapillarensystem zu einem Elektrodensystem geführt wird. Für moderne Teststreifen genügt dabei eine Blutmenge von ca. 1,5 Mikrolitern, teilweise auch Blutmengen unter einem Mikroliter. Bei dem Elektrodensystem kann es sich z. B. um Goldelektroden handeln, welche mit einer Beschichtung versehen sind. Die Beschichtung enthält zumeist verschiedene Enzyme und so genannte Mediatoren und bewirkt, dass sich innerhalb der Probe an den Elektroden Ladungsträger (beispielsweise in Form von Redoxmolekülen) bilden, deren Konzentration abhängig ist von der Blutglukosekonzentration. Die Konzentration dieser Ladungsträger kann mittels der Goldelektroden und einem geeigneten, dem Fachmann bekannten Messsystem beispielsweise mittels einer vergleichsweise einfachen Strom-Spannungs-Messung bestimmt werden und so auf die Blutglukosekonzentration zurückgerechnet werden.

Ein wesentliches Element derartiger portabler Diagnosesysteme bilden somit die entsprechenden Teststreifen bzw. Systeme zur Ausgabe dieser Teststreifen. Typischerweise werden von einem Diabetiker ca. 5 bis 7 derartiger Teststreifen pro Tag benötigt. Essentiell ist dabei, dass die Teststreifen sauber und trocken aufbewahrt werden, um nicht durch eine entsprechende Verschmutzung bzw. durch Einwirkung von Feuchtigkeit die Messung der Blitglukosekonzentration zu verfälschen.

WO 03/082091 A2 beschreibt ein integriertes Probenmessgrät, umfassend eine Lanzette, einen Sensor und eine Testreifenkartusche, angeordnet in einem Gehäuse. Das Testsystem gibt automatisch einen Testsheifen aus und positioniert diesen relativ zu einer Einstishstelle.

Die EP 1 321 769 A1 offenbart ein Testsystem mit Mitteln zum Aufbewahren und Ausgeben von Teststreifen. Die beschriebene Vorrichtung offenbart im Wesentlichen eine feuchtigkeits- und luftdichte Anordnung, in welcher entsprechende Teststreifen gelagert und mittels einer Schiebevorrichtung ausgegeben werden können. Die beschriebene Vorrichtung enthält jedoch selbst keine Mittel zur Diagnose, d.h. beispielsweise zur Messung der Blutglukosekonzentration. Weiterhin enthält die beschriebene Vorrichtung keine Mittel zur Bereitstellung entsprechender Proben, wie beispielsweise ein Lanzettensystem zur Gewinnung von Blutstropfen. Somit ist die in der EP 1 321 769 A1 beschriebene Vorrichtung nur in Kombination mit entsprechenden Lanzettensystemen und Diagnosesystemen zur Auswertung der Teststreifen einsetzbar.

Auch in der WO 02/055008 A2 wird eine Vorrichtung zum Aufbewahren und Ausgeben von Teststreifen offenbart. Dabei wird eine Teststreifenkassette in einer Röhre aufbewahrt, welche wiederum in einem Gehäuse untergebracht ist. Das Gehäuse weist einen Knopf und einen Mechanismus auf, mittels derer ein Teststreifen aus dem Röhrchen ausgegeben werden kann. Auch die WO 02/055008 beschreibt kein vollständiges Diagnosesystem, sondern lediglich eine entsprechende Ausgabeeinheit für Teststreifen, welche nur in Kombination mit anderen Systemen, insbesondere einem Lanzettensystem und einem Diagnose- bzw. Messsystem einsetzbar ist. Hierdurch wird der Platzbedarf für die für eine Routinekontrolle der Blutglukosekonzentration erforderlichen Vorrichtungen stark erhöht.

Die WO 03/083469 A2 offenbart ein integriertes Testgerät, welches beispielsweise für eine elektrochemische oder photometrische Analyse einer Blutprobe eingesetzt werden kann. Das integrierte Messsystem schließt ein Lanzettensystem, einen Sensor und eine Teststreifenpatrone in einem einzigen Gehäuse ein. Das in der WO 03/083469 A2 beschriebene System weist jedoch verschiedene für den täglichen Gebrauch entscheidende Nachteile auf. Insbesondere handelt es sich bei dem beschriebenen System um ein einstückiges System, welche alle Komponenten in einem Gehäuse integriert. Dadurch ist das beschriebene System sehr raumaufwändig und schwer. Auch ist bei dem beschriebenen System ein separates Spannen des integrierten Lanzettensystems erforderlich. Weiterhin löst das in der WO 03/083469 A2 beschriebene System nicht das bei Blutglukosekonzentrationsmessungen häufig auftretende Problem, dass die Eigenschaften der verwendeten Teststreifen von Charge zu Charge der Teststreifen variieren können. Hierdurch ergeben sich teilweise massive Schwankungen der Genauigkeit der Blutglukosekonzentrationsmessung. Weiterhin weist das in der WO 03/083469 A2 beschriebene System den Nachteil auf, dass eine zusätzliche Teststreifenpatrone erforderlich ist, welche in das System eingefügt werden muss, um entsprechende Teststreifen nachzuladen. Durch dieses "doppelte Gehäuse" wird der Platzbedarf für das beschriebene System zusätzlich erhöht.

### Aufgabe

Es ist daher eine Aufgabe der vorliegenden Erfindung, ein System bereitzustellen, welches die beschriebenen Nachteile des Standes der Technik vermeidet. Insbesondere soll ein System zur Verfügung gestellt werden, welches einfach zu handhaben ist, unanfällig ist gegenüber Störungen und Bedienfehlern und einen geringen Platzbedarf aufweist.

### Lösung

Diese Aufgabe wird durch die Erfindung mit den Merkmalen des unabhängigen Anspruchs gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt dieser Beschreibung gemacht.

Es wird ein Diagnosesystem zum Ermitteln von Stoffkonzentrationen in flüssigen Proben, insbesondere zur Blutzuckermessung im menschlichen Blut, vorgeschlagen. Weiterhin wird ein Magazinsystem zur Verwendung in einem erfindungsgemäßen Diagnosesystem sowie ein Verfahren zum Bedienen eines erfindungsgemäßen Diagnosesystems vorgeschlagen. Neben dem erwähnten Einsatz des erfindungsgemäßen Diagnosesystems zur Messung von Blutglukosekonzentrationen ist auch ein anderweitiger Einsatz des beschriebenen Diagnosesystems denkbar, beispielsweise zur schnellen und einfachen Analyse bestimmter Proben, beispielsweise Lebensmitteln. Anstelle einer Blutglukosekonzentrationsmessung kann dabei beispielsweise eine Messung anderer Parameterdurchgeführt werden.

Das Diagnosesystem soll ein Diagnosemodul aufweisen sowie ein in das Diagnosemodul integriertes Lanzettensystem. Unter einem Lanzettensystem kann dabei ein beliebiges System zum Bereitstellen einer flüssigen Probe verstanden werden, insbesondere ein Nadelsystem zu Einstechen einer Nadel in eine Probe. Im Normalfall wird es sich dabei um ein mit einer Lanzette ausgestattetes Lanzettensystem zum Gewinnen eines Blutstropfens handeln. Dabei kann beispielsweise die Lanzette auswechselbar sein und einfach oder mehrfach benutzbar sein. Verschiedene derartige Systeme sind aus dem Stand der Technik bekannt, insbesondere auch Systeme, welche zum Spannen ein Drehschiebergetriebe aufweisen (siehe z. B. US 6,419,661 B1). Ein Spannen derartiger Lanzettensysteme kann auf verschiedene Weise erfolgen, beispielsweise über eine Linearbewegung und/oder über eine Drehbewegung. Es sind jedoch auch andere Lanzettensysteme sinngemäß einsetzbar, beispielsweise Lanzettensysteme oder Probenentnahmesysteme zum Entnehmen von Lebensmittelproben.

Weiterhin weist das Diagnosesystem ein von dem Diagnosemodul getrenntes Magazinmodul zum Speichern von Teststreifen auf. Dabei kann es sich beispielsweise um einen der oben erwähnten Teststreifen für eine elektrochemische oder photometrische Analyse flüssiger Proben handeln. Insbesondere kann es sich dabei um Teststreifen der oben beschriebenen Art mit einem Kapillarsystem und einer oder mehreren Elektroden sowie zusätzlichen chemischen Hilfsstoffen (z.B. Enzyme, Mediatoren) handeln. Unter Teststreifen lassen sich sinngemäß auch andere Testvehikel verstehen, insbesondere kleine Teströhrchen oder kontinuierliche Rollen von Teststreifen.

Das Diagnosesystem weist weiterhin eine Verbindungsvorrichtung auf, mittels derer das Magazinmodul mit dem Diagnosemodul verbindbar ist.

Dabei kann die Verbindungsvorrichtung eine Spannvorrichtung zum Spannen des Lanzettensystems aufweisen. Alternativ kann die Verbindungsvorrichtung auch mit einer derartigen Spannvorrichtung zum Spannen des Lanzettensystems verbunden sein, derart, dass bei der Verbindung des Magazinmoduls mit dem Diagnosemodul das Lanzettensystem gespannt wird.

Alternativ oder zusätzlich kann die Verbindungsvorrichtung auch eine Betätigungsvorrichtung zum Bedienen des Magazinmoduls, insbesondere zur Ausgabe mindestens eines Teststreifens, aufweisen und/oder mit einer derartigen Betätigungsvorrichtung verbunden sein. Bei dieser Alternative der Erfindung wird bei der Verbindung des Magazinmoduls mit dem Diagnosemodul mindestens ein Teststreifen ausgegeben, wobei unter "Ausgeben" beispielsweise ein Vorschub eines Teststreifens in eine Messposition verstanden werden kann, insbesondere in eine Messposition, in der eine flüssige Probe (beispielsweise ein Blutstropfen) aufgenommen werden kann.

Das beschriebene Diagnosesystem weist gegenüber den herkömmlichen, aus dem Stand der Technik bekannten Vorrichtungen zahlreiche Vorteile auf. Insbesondere sind beim Transport des beschriebenen Diagnosesystems, solange das Diagnosesystem nicht für den Einsatz vorbereitet wird, das Diagnosemodul und das Magazinmodul getrennt Somit lassen sich beide Module getrennt aufbewahren, wodurch das System im zerlegten Zustand Platz sparender verpackt werden kann. Somit muss beispielsweise ein Diabetiker nicht beide Module gleichzeitig beispielsweise in einer Tasche (z.B. einer Hosentasche) transportieren, sondern kann die beiden Module getrennt verpacken und getrennt mit sich führen.

Weiterhin bewirkt die Trennung von Magazinmodul und Diagnosemodul beispielsweise im Vergleich zur WO 03/083469 A2, dass die Versorgung des Diagnosesystems mit Teststreifen stets zuverlässig geregelt ist Dabei ist keine zusätzliche raumaufwändige Teststreifentrommel erforderlich, welche erst in das Diagnosesystem eingefügt werden müsste. Zum Nachfüllen von Teststreifen kann einfach das gesamte Magazinmodul ausgetauscht werden. Beispielsweise können komplette Magazinmodule im Handel erhältlich sein, eventuell sogar im Rahmen eines entsprechenden Pfandsystems. Eine doppelwandige Verpackung der Teststreifen, wie sie beispielsweise in der WO 03/083469 A2 vorgesehen ist, kann entfallen.

Weiterhin ist von Vorteil, dass beim Zusammenfügen des Magazinmoduls und des Diagnosemoduls das Lanzettensystem unmittelbar gespannt wird. Es kann beispielsweise auch eine Vorrichtung vorgesehen sein, welche bei einem Trennen des Magazinmoduls vom Diagnosemodul das Lanzettensystem wieder entspannt. Insgesamt kann daher das gesamte System zerlegt, im entspannten Zustand transportiert werden, wobei beim Zusammenfügen der beiden Module das Lanzettensystem automatisch gespannt werden kann, so dass das System nach dem Zusammenfügen sofort betriebsbereit ist.

Das System kann vorteilhafterweise dahingehend erweitert werden, dass eine Datenaustauschvorrichtung zum Austausch von Chargeninformationen der Teststreifen zwischen dem Magazinmodul und dem Diagnosemodul vorgesehen ist. Bei diesen Chargeninformationen kann es sich, wie oben beschrieben, um Informationen darüber handeln, wie die Teststreifen von einer vorgegebenen Norm abweichen, bzw. wie aus den mittels dieser Charge der Teststreifen gewonnenen Messinformationen beispielsweise eine Blutglukosekonzentration berechnet werden kann. Bei diesen Chargeninformationen kann es sich beispielsweise um Parameter einer entsprechenden Anpassungsfunktion handeln.

Insbesondere kann die Datenaustauschvorrichtung einen im Magazinmodul angeordneten Transponder und ein im Diagnosemodul angeordnetes Transponderlesegerät aufweisen. Dabei kann beispielsweise, wenn das Magazinmodul mit dem Diagnosemodul verbunden wird, automatisch der im Magazinmodul angeordnete Transponder von dem im Diagnosemodul angeordneten Transponderlesegerät angeregt werden, entsprechende Chargeninformationen der Teststreifen an das Transponderlesegerät zu übermitteln. Dabei kann es sich um kommerziell erhältliche Transponder und Transponderlesegeräte handeln. Dieser Datenaustausch kann beispielsweise automatisch erfolgen, sobald das Magazinmodul mit dem Diagnosemodul verbunden wird. Dieser automatische Austausch von Chargeninformationen hat den Vorteil, dass die Wahrscheinlichkeit, dass Fehler beim Übertragen der Chargeninformationen auftreten, drastisch reduziert wird. Bei kommerziellen Systemen müssen Chargeninformationen in das Diagnosemodul zumeist manuell eingegeben werden oder mittels eines speziellen, vom Anwender durchzuführenden Lesevorgangs. Diese manuelle Eingabe bzw. der vom Anwender vorzunehmende Lesevorgang ist jedoch äußerst fehlerträchtig und kann dazu führen, dass entweder eine Übertragung der Chargeninformationen vollständig vergessen wird und somit unterbleibt, oder dass Chargeninformationen fehlerhaft eingetragen werden. Diese Fehler können fatale Folgen haben, dahingehend, dass beispielsweise falsche Blutglukosekonzentrationen berechnet werden, wodurch vollständig falsche medizinische Gegenmaßnahmen, beispielsweise in Form falscher Dosierungen von Insulin, angeregt werden.

Alternativ oder zusätzlich kann die Datenaustauschvorrichtung auch einen im Magazinmodul angeordneten Datenspeicher, insbesondere ein EPROM, aufweisen. Dieser EPROM wird häufig auch als "ROM-Key" bezeichnet. Diese Weiterbildung ist insbesondere in Kombination mit einer weiteren Ausgestaltung sinnvoll, bei welcher die Datenaustauschvorrichtung mindestens einen elektrischen Kontakt aufweist. Dieser elektrische Kontakt kann beispielsweise in der Verbindungsvorrichtung angeordnet sein. Auf diese Weise kann beispielsweise der im Magazinmodul angeordnete Datenspeicher direkt mittels elektrischer Kontakte ausgelesen werden. Dies ist insbesondere dann von Vorteil, wenn eine drahtlose Datenübertragung, beispielsweise mittels eines Transponders, nicht gewünscht ist. Drahtlose Datenübertragungen können für viele Einsatzgebiete störanfällig sein, da beispielsweise auch Störsignale benachbarter Geräte mit aufgenommen werden können. So kann es beispielsweise sein, wenn zwei Diabetiker in unmittelbarer Nähe entsprechende Geräte einsetzen, dass Daten von einem Gerät auf unerwünschte Weise vom anderen Gerät wahrgenommen werden. Dies kann, wie oben beschrieben, fatale Folgen haben. In der beschriebenen Weiterbildung wird hingegen bei Verbindung des Magazinmoduls mit dem Diagnosemodul automatisch eine sichere und störungsunanfällige Übertragung der entsprechenden Chargeninformationen durchgeführt, ohne dass es eines weiteren Eingriffs durch den Benutzer bedarf. Trotz der beschriebenen Nachteile können zusätzlich oder alternativ auch Transponder eingesetzt werden.

Die Verbindungsvorrichtung zur Verbindung des Magazinmoduls mit dem Diagnosemodul kann verschiedenartig ausgestaltet sein. So kann diese Verbindungsvorrichtung beispielsweise eine Einschubvorrichtung aufweisen. Bei einer derartigen Einschubvorrichtung, welche beispielsweise eine lineare Führung aufweisen kann, führt der Benutzer beim Zusammenfügen von Magazinmodul und Diagnosemodul eine lineare Verschiebung beider Module gegeneinander durch. Diese lineare Verschiebung kann insbesondere dazu genutzt werden, das Lanzettensystem zu spannen. Dies ist dadurch bedingt, dass viele Lanzettensysteme ein oder mehrere linear wirkende Federsysteme aufweisen, welche mittels der genannten linearen Verschiebung mit einer Spannung beaufschlagt werden können (siehe z. B. auch US 6,419,661 B1). Insbesondere kann die Einschubverbindung eine Schwalbenschwanzführung aufweisen. Eine derartige Verbindung zwischen Magazinmodul und Diagnosemodul ist mechanisch besondere stabil.

Weiterhin kann das Diagnosesystem derart ausgestaltet sein, dass die Spannvorrichtung einen mit einem Federsystem verbundenen Spannschlitten aufweist. Dieser Spannschlitten kann beispielsweise in dem Diagnosemodul angeordnet sein. Weist die Verbindungsvorrichtung, wie oben, eine Einschubvorrichtung auf, beispielsweise eine Schwalbenschwanzführung, so kann beim Zusammenfügen des Magazinmoduls und des Diagnosemoduls durch eine Linearbewegung beispielsweise der in dem Diagnosemodul angeordnete Spannschlitten ergriffen und mit einer Bewegung des Magazinmoduls relativ zum restlichen Diagnosemodul linear verschoben werden, wobei das Federsystem gespannt wird. Eine entsprechende Rastvorrichtung, beispielsweise ein einfacher Haken, kann dann das Federsystem in gespanntem Zustand halten, solange bis das Lanzettensystem ausgelöst wird, wobei der Haken gelöst und das Federsystem freigegeben wird und auf eine Lanzette einwirkt. Derartige Auslösevorrichtungen sind aus dem Stand der Technik bekannt und sollen daher hier nicht näher beschrieben werden (siehe z. B. auch US 6,419,661 B1).

Als besonders vorteilhaft hat es sich erwiesen, wenn das Diagnosemodul ein Computersystem und/oder ein Display aufweist. Das Computersystem kann verschiedene Aufgaben übernehmen: Insbesondere kann das Computersystem derart ausgestaltet sein, dass es direkt oder indirekt über entsprechende Messdatenerfassungssysteme die Messdaten zum Ermitteln von Stoffkonzentrationen erfasst und entsprechend auswertet. Dabei können verschiedene Teilsysteme eingeschlossen sein, wie beispielsweise Datenspeicher, insbesondere flüchtige und nicht-flüchtige Speicher. Weiterhin kann das Computersystem auch entsprechende Hardwarekomponenten zum Erfassen und Austauschen der Chargeninformationen aufweisen. So kann beispielsweise ein Transponderlesegerät in das Computersystem integriert sein. Bei dem Computersystem kann es sich beispielsweise um einen Mikrocomputer handeln. Weiterhin kann das Computersystem eine Vorrichtung zum Ausgeben der Daten aufweisen. Beispielsweise kann das Computersystem mit einem Display in Verbindung stehen, über welches Sichtinformationen an den Benutzer des Diagnosesystems weitergegeben werden, insbesondere Informationen über die ermittelte Blutglukosekonzentration.

In einer weiteren vorteilhaften Ausgestaltung weist das Diagnosesystem insbesondere eine Betätigungsvorrichtung zum Bedienen des Magazinmoduls, eine Auslösevorrichtung zum Auslösen des Lanzettensystems und ein Bediensystem zum Bedienen des Diagnosemoduls auf. Die Betätigungsvorrichtung, die Auslösevorrichtung und das Bediensystem können dabei getrennte Bedienelemente bilden oder auch in einem einzelnen Bedienelement realisiert bzw. integriert sein. Die Betätigungsvorrichtung, die Auslösevorrichtung und das Bediensystem können dabei am Magazinmodul und/oder auch am Diagnosemodul vorgesehen sein. Auch eine Verteilung auf das Diagnosemodul und das Magazinmodul ist denkbar, beispielsweise dahingehend, dass ein oder mehrere Bedienelemente am Diagnosemodul und ein oder mehrere Bedienelemente am Magazinmodul vorgesehen sind.

Das Diagnosesystem kann insbesondere derart ausgestaltet sein, dass es ein vertikales Griffstück und eine im Wesentlichen horizontale Bedienoberfläche aufweist. Insbesondere kann die Bedienoberfläche mindestens ein Bedienelement aufweisen. So kann insbesondere das Magazinmodul als Griffstück ausgestaltet sein, wohingegen das Diagnosemodul die horizontale Bedienoberfläche aufweist. Beispielsweise kann das aus dem Magazinmodul und dem Diagnosemodul zusammengesetzte Diagnosesystem im zusammengesetzten Zustand die Form eines T bzw. einer Pistole aufweisen. Auf diese Weise ist eine gute Handhabbarkeit des Diagnosesystems gewährleistet. Insbesondere ist es dabei von Vorteil, wenn das gesamte Diagnosesystem mit einer Hand betätigbar ist Dabei können beispielsweise mindestens drei Bedienelemente bei mit dem Diagnosemodul verbundenem Magazinmodul mit den Fingern einer Hand bedienbar sein. So kann beispielsweise eine Betätigungsvorrichtung zum Bedienen des Magazinmoduls im Magazinmodul vorgesehen sein, welche mit dem Zeigefinger, dem Daumen oder dem Handballen einer das Magazinmodul umschließenden menschlichen Hand bedient werden kann. Weiterhin kann eine Auslösevorrichtung, z.B. in Form eines Druckknopfes, im Diagnosemodul vorgesehen sein, welche z. B. mit dem Daumen einer das Magazinmodul umfassenden Hand betätigt werden kann und das Lanzettensystem auslöst. Auch eine Auslösevorrichtung am Magazinmodul, beispielsweise in Form eines mit einem Zeigefinger einer das Magazinmodul umschließenden Hand betätigbaren "Abzugs" ist denkbar. Alternativ kann die Auslösevorrichtung auch derart ausgestaltet sein, dass z. B. beim Aufdrücken eines Lanzettensystems (beispielsweise einer federnd gelagerten Lanzettenkappe) durch den Druck das Lanzettensystem automatisch ausgelöst wird, wobei keine zusätzliche Auslösevorrichtung erforderlich ist Weiterhin können eine oder mehrere Druckknöpfe auf dem Diagnosemodul vorgesehen sein, welche als Bediensystem zum Bedienen des Diagnosemoduls wirken. Auch diese können beispielsweise so angeordnet sein, dass sie mit dem Daumen einer das Magazinmodul umschließenden Hand bedienbar sind. Dieses Bediensystem kann beispielsweise das Computersystem zurücksetzen, initialisieren oder einen Messvorgang starten.

Wie oben beschrieben, lassen sich mit dem erfindungsgemäßen Diagnosesystem verschiedene Teststreifen und dem Fachmann bekannte Testverfahren einsetzen, beispielsweise elektrochemische oder photometrische Testverfahren. Als vorteilhaft hat es sich erwiesen, wenn Teststreifen verwendet werden, welche, wie oben beschrieben, ein Kapillarsystem sowie mehrere integrierte Elektroden aufweisen. Zusätzlich können, wie ebenfalls oben beschrieben, die Elektroden beispielsweise mit Enzymen oder Mediatoren beschichtet sein, um, bei Anwesenheit von Blutglukose, freie Ladungsträger zu generieren. Es hat sich daher als vorteilhaft erwiesen, wenn das Diagnosesystem im Magazinmodul mindestens eine Messelektrode aufweist, wobei bei mit dem Diagnosemodul verbundenem Magazinmodul das Diagnosemodul über die mindestens eine Messelektrode mit mindestens einem Teststreifen verbindbar ist. Unter verbindbar ist dabei eine Ausgestaltung zu verstehen, bei der beispielsweise in einer Position des Teststreifens Elektroden im Teststreifen mit der mindestens einen Messelektrode verbunden sind.

Das Magazinsystem kann vorteilhafterweise insbesondere als Stapelmagazin für Teststreifen ausgestaltet sein. Es sind jedoch auch andere Ausgestaltungen denkbar, beispielsweise Rollenmagazine oder andere Systeme. Vorteilhafterweise weist dabei das Magazinsystem eine Betätigungsvorrichtung zum Ausgeben von Teststreifen auf. Bei dieser Betätigungsvorrichtung kann es sich beispielsweise um einen Hebel oder einen Druckknopf, z.B. in Form eines Abzugs, handeln. Diese Betätigungsvorrichtung ist vorteilhafterweise so ausgestaltet, dass sie einen Betätigungsknopf, eine Schubstange und ein Schubstück zum Verschieben eines Teststreifens aufweist. Dabei kann das Schubstück insbesondere zwei Schubkanten zum Erfassen eines Teststreifens aufweisen: eine erste Schubkante (Messkante) und eine zweite Schubkante (Auswurfkante). Die Betätigungsvorrichtung kann dann insbesondere so ausgestaltet sein, dass bei einer ersten Betätigung des Betätigungsknopfs ein Teststreifen mit der ersten Schubkante (Messkante) erfasst und in eine Messposition geschoben wird. Insbesondere kann der Teststreifen in dieser Messposition von den oben genannten Messelektroden kontaktiert werden, so dass in dieser Position eine Messung einer Stoffkonzentration, beispielsweise eine Blutglukosekonzentrationsmessung, durchgeführt werden kann. Anschließend kann der Betätigungsknopf dann beispielsweise ein zweites Mal betätigt werden, wobei derselbe Teststreifen von der zweiten Schubkante (Auswurfkante) des Schubstücks erfasst wird und aus dem Magazinmodul, beispielsweise über einen Ausgabeschlitz, ausgeworfen wird. Der Teststreifen kann dann problemlos entsorgt werden. Anstelle von "Kanten" lassen sich sinngemäß auch andere Einrichtungen einsetzen, welche vergleichbare Wirkung aufzeigen, beispielsweise entsprechende Greifer, Stifte, Nuten oder Ähnliches. Das beschriebene System hat insbesondere den Vorteil, dass ein Benutzer zu keinem Zeitpunkt der Diagnose den Teststreifen mit der Hand berühren muss, wodurch die Gefahr, dass Teststreifen beispielsweise durch Fingerschweiß oder Verunreinigungen auf der Handfläche verunreinigt werden, stark verringert wird.

Weiterhin hat es sich als vorteilhaft erwiesen, wenn das Magazinmodul weitgehend luftdicht ausgestaltet ist bzw. so ausgestaltet ist, dass Verunreinigungen und Feuchtigkeit, z.B. Luftfeuchtigkeit, weitgehend von den im Magazinmodul gelagerten Teststreifen ferngehalten werden. Insbesondere kann das Magazinmodul auch ein Trockenmittel zur Verringerung der Luftfeuchtigkeit im Magazinmodul aufweisen.

Für die Bedienung mit einer Hand hat es sich als vorteilhaft erwiesen, wenn der Ort der Bereitstellung einer flüssigen Probe, insbesondere der Ort eines Einstechens mittels einer Lanzette (z. B. ein Lanzettenloch in einer Lanzettenkappe des Diagnosesystems), und der Ort einer Aufnahme der flüssigen Probe, insbesondere der Ort der Aufgabe eines Blutstropfens auf einen Teststreifen in Messposition, eng benachbart sind. Somit ist nur eine kleine laterale Bewegung des Diagnosesystems erforderlich, um nach dem Einstechen des Lanzettensystems und dem Erzeugen eines Blutstropfens diesen mit dem Teststreifen in Messposition aufzunehmen, was ergonomisch besonders günstig ist. Zu diesem Zeck kann das Diagnosesystem beispielsweise derart ausgestaltet sein, dass es ein Lanzettenloch und einen Ausgabeschlitz für Teststreifen aufweist, welche vorteilhafterweise nicht weiter als 50 mm, bevorzugt nicht weiter als 40 mm und besonders bevorzugt in einem Bereich zwischen 8 mm und 35 mm voneinander beabstandet sind.

Die oben beschriebene "T-Form" bzw. "Pistolenform" eines aus einem Magazinmodul und einem Diagnosemodul zusammensetzbaren Diagnosesystems kann erfindungsgemäß auch ohne das beschriebene automatische Spannen eines Lanzettensystems bzw. die automatische Ausgabe eines Teststreifens beim Verbinden von Magazinmodul und Diagnosemodul eingesetzt werden. Dabei kann, wie oben beschrieben, das Diagnosesystem ein im Wesentlichen vertikales Griffstück und eine im Wesentlichen horizontale Bedienoberfläche aufweisen. Insbesondere kann die Bedienoberfläche eine Oberfläche des Diagnosemoduls sein. Die Verteilung von Bedienelementen und die Funktionalität der einzelnen Komponenten des Diagnosesystems können jeweils wie oben beschrieben ausgestaltet sein. Dieses zerlegbare "T-förmige" bzw. "pistolenförmige" Diagnosesystem zeichnet sich gegenüber aus dem Stand der Technik bekannten Systemen durch stark verbesserte ergonomische Eigenschaften und verbesserte Handhabbarkeit aus, wobei gleichzeitig durch die Zerlegbarkeit verhindert wird, dass die sperrige "T-Gestalt" des Diagnosesystems die Transporteigenschaften verschlechtert.

Im Folgenden wird die Erfindung anhand von Ausführungsbeispielen näher beschrieben. Die-Erfindung ist jedoch nicht auf die dargestellten Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: eine Zusammenbauzeichnung eines Magazinmoduls und eines Diagnosemoduls zu einem erfindungsgemäßen Diagnosesystem;
- Figur 2: eine perspektivische Darstellung eines Magazinmoduls;
- Figur 3: eine perspektivische Darstellung des Magazinmoduls gemäß Figur 2 mit eingedrücktem Betätigungsknopf;
- Figur 4: eine Schnittdarstellung eines Magazimnoduls gemäß Figur 2;
- Figur 5: eine Schnittdarstellung des Magazinmoduls gemäß Figur 4 mit eingedrücktem Betätigungsknopf;
- Figur 6: eine Schnittdarstellung eines Diagnosemoduls vor Befestigen eines Magazinmoduls;
- Figur 7: eine Schnittdarstellung der Anordnung gemäß Figur 6 nach Befestigen des Magazinmoduls;
- Figur 8: einen Spannschlitten für ein Lanzettensystem in perspektivischer Darstellung;
- Figur 9A: eine perspektivische Darstellung eines zu Figur 1 alternativen Diagnosesystems in Stapelform in Rückansicht;
- Figur 9B: eine perspektivische Darstellung des Diagnosesystems gemäß Figur 9A in Vorderansicht;
- Figur 10A: eine perspektivische Darstellung eines zu den Figuren 1 und 9A und 9B alternativen Diagnosesystems in Reihenform in Rückansicht;
- Figur 10B: eine perspektivische Darstellung des Diagnosesystems gemäß Figur 10A in Vorderansicht; und
- Figur 11: einen Ablaufplan eines Beispiels eines erfindungsgemäßen Verfahrens zum Bedienen eines erfindungsgemäßen Diagnosesystems.

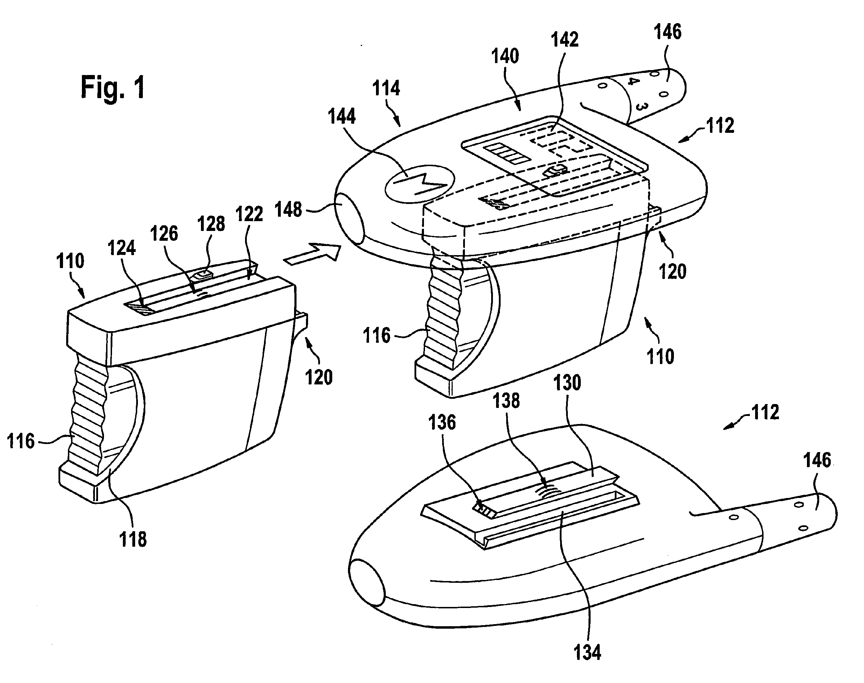

In Figur 1 ist eine perspektivische Darstellung des Zusammenfügens eines Magazinmoduls 110 und eines Diagnosemoduls 112 zu einem Diagnosesystem 114 dargestellt. Dabei ist das Diagnosemodul einmal in Ansicht von unten (Darstellung rechts unten) und von oben (im zusammengebauten Zustand rechts oben) dargestellt.

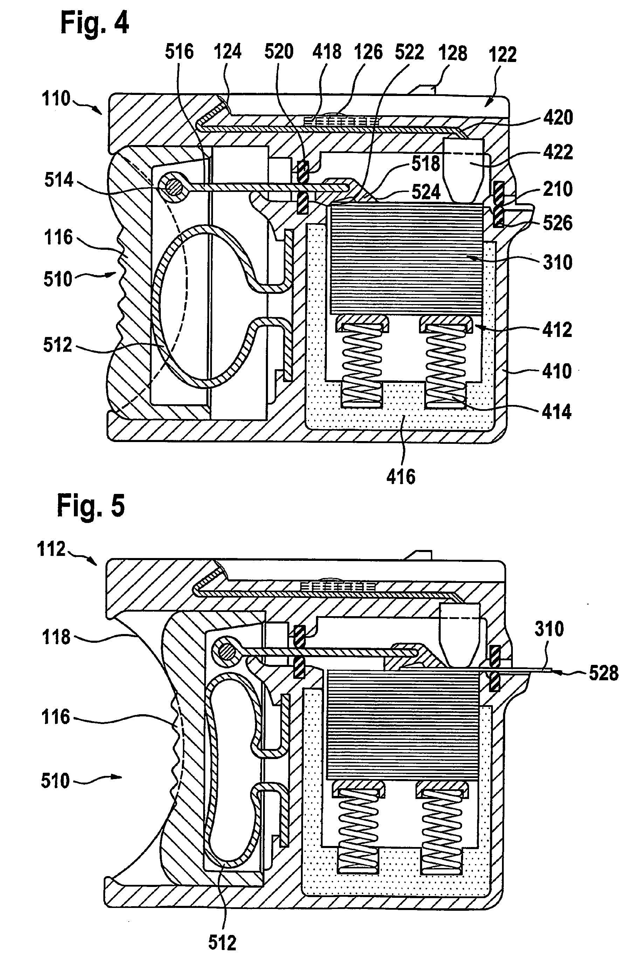

Das Magazinmodul 110 ist in diesem bevorzugten Ausführutigsbeispiel als Griffstück ausgeführt, welches beispielsweise mit einer Hand umschlossen werden kann. Das Magazinmodul 110 weist an einer Stirnseite einen Betätigungsknopf 116 als Teil einer Betätigungsvorrichtung zum Ausgeben von Teststreifen aus dem Magazinmodul (siehe z. B. Bezugsziffer 510 in Fig. 5 unten) auf. Dieser Betätigungsknopf 116 ist in einer Ausbuchtung 118 in einer Stirnseite des Magazinmoduls 110 aufgenommen. Die Funktionsweise des Betätigungsknopfs 116 und der Betätigungsvorrichtung 510, deren Bestandteil der Betätigungsknopf 116 bildet, wird weiter unten im Detail beschrieben. Die Oberfläche des Betätigungsknopfs 116 ist in diesem Ausführungsbeispiel geriffelt dargestellt, um die Griffigkeit des Betätigungsknopfs 116 zu erhöhen. Wird das Magazinmodul 110 mit einer Hand umschlossen, so kann beispielsweise der Betätigungsknopf 116 mit dem Daumen der Hand oder mit dem Handballen betätigt werden. Weiterhin weist das Magazinmodul 110 eine Ausgabeeinheit 120 auf, über die bei Betätigung der Betätigungsvorrichtung 510, insbesondere des Betätigungsknopfs 116, in dem Magazinmodul 110 enthaltene Teststreifen 310 (siehe z. B. Figur 5 unten) ausgegeben bzw. in eine Testposition geschoben werden können.

An seiner Oberseite weist das Magazinmodul 110 eine schwalbenschwanzförmige Nut 122 auf. Diese schwalbenschwanzförmige Nut 122 bildet eine magazinseitige Führungsschiene. In der schwalbenschwanzförmigen Nut 122 sind zwei Gruppen von magazinseitigen elektrischen Kontakten 124, 126 aufgenommen: Zum einen eine Gruppe von (in diesem Fall drei) Messelektrodenkontakten 124 am geschlossenen stirnseitigen Ende der schwalbenschwanzförmigen Nut 122 und zum anderen eine Gruppe von (in diesem Ausfuhrungsbeispiels ebenfalls drei) in der Mitte der schwalbenschwanzförmigen Nut 122 angeordneten magazinseitigen ROM-Key-Kontakten 126. Die Funktion dieser Kontakte 124, 126 wird weiter unten beschrieben.

Weiterhin weist das Magazinmodul 110 in diesem Ausführungsbeispiel eine magazinseitige Mitnehmernase 128 auf, welche neben der schwalbenschwanzförmigen Nut 122 auf der Oberseite des Magazinmoduls 110 angeordnet ist.

Das Diagnosemodul 112 weist an seiner Unterseite eine diagnosemodulseitige Schwalbenschwanzschiene 130 auf, welche in die magazinseitige schwalbenschwanzförmige Nut 122 eingeschoben werden kann, ohne dass dabei ein nennenswertes Spiel auftritt. Durch Einschieben der diagnosemodulseitigen Schwalbenschwanzschiene 130 in die magazinseitige schwalbenschwanzförmige Nut 122 entsteht also eine feste Verbindung zwischen Diagnosemodul 112 und Magazinmodul 110. Die diagnosemodulseitige Schwalbenschwanzschiene 130 ist montiert auf eine Montageplatte 132 auf der Unterseite des Diagnosemoduls 112. Diese Montageplatte 132 weist einen Mitnehmerschlitz 134 auf, in welchen bei auf das Magazinmodul 110 montiertem Diagnosemodul 112 die Mitnehmernase 128 auf der Oberseite des Magazinmoduls 110 eingreifen kann.

Weiterhin weist die diagnosemodulseitige Schwalbenschwanzschiene 130 zwei Gruppen von Kontakten 136, 138 auf: Zum einen eine Gruppe von (in diesem Ausführungsbeispiel drei) diagnosemodulseitigen Messelektrodenkontakten 136 an einer Stirnseite der diagnosemodulseitigen Schwalbenschwanzschiene 130 und zum anderen eine Gruppe von (in diesem Fall ebenfalls drei) diagnosemodulseitigen ROM-Key-Kontakten 138 in der Mitte der diagnosemodulseitigen Schwalbenschwanzschiene 130. Die diagnosemodulseitigen Messelektrodenkontakte 136 und ROM-Key-Kontakte 138 sind dabei so angeordnet, dass bei einem Einschieben der diagnosemodulseitigen Schwalbenschwanzschiene 130 in die schwalbenschwanzförmige Nut 122 des Magazinmoduls 110 die diagnosemodulseitigen Messelektrodenkontakte 136 auf den magazinseitigen Messelektrodenkontakten 124 und die diagnosemodulseitigen ROM-Key-Kontakte 138 auf den magazinseitigen ROM-Key-Kontakten 126 zu liegen kommen und entsprechende elektrische Verbindungen zwischen dem Magazinmodul 110 und dem Diagnosemodul 112 herstellen.

Weiterhin weist das Diagnosemodul 112 bei zusammengesetztem Diagnosesystem 114 eine im Wesentlichen horizontale Bedienoberfläche 140 auf. In die Bedienoberfläche 140 ist ein Display 142, insbesondere in diesem Ausführungsbeispiel ein LCD-Display, eingelassen. Über dieses Display 142 können dem Benutzer des Diagnosesystems 114 Informationen übermittelt werden, beispielsweise Messergebnisse einer Blutglukosekonzentrationsmessung, sowie andere Information, wie beispielsweise ein Batteriezustand. Das Display 142 kann alternativ beispielsweise auch als Touch-Screen ausgestaltet sein, wobei eine entsprechende Eingabe bzw. Bedienung des Diagnosemoduls 112 bzw. Diagnosesystems 114 über ein Berühren entsprechender Oberflächenbereich auf dem Touch-Screen erfolgen kann.

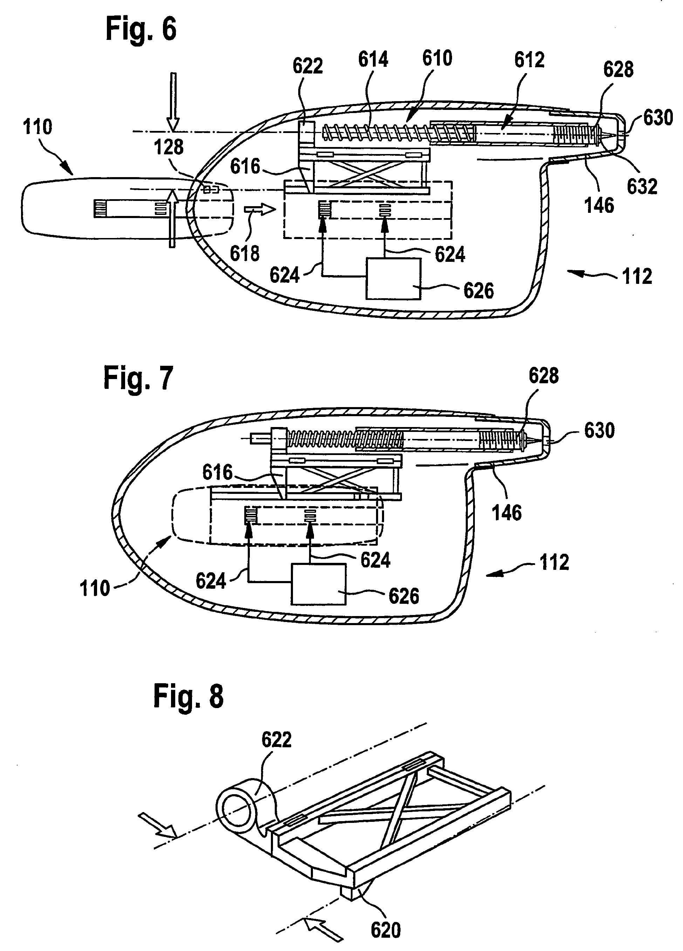

Weiterhin weist die Bedienoberfläche 140 einen Bedienknopf 144 auf, welcher bündig in die Bedienoberfläche 140 eingelassen ist und mittels dessen wesentliche Funktionen des Diagnosesystems 114 bedient werden können. Beispielsweise kann mittels dieses Bedienknopfs 144 ein in dem Diagnosemodul 112 enthaltenes Computersystem (siehe z. B. Bezugsziffer 626 in den Figuren 6 und 7 unten) initialisiert werden oder eine Messung gestartet werden. Auch als Ein- und Ausschaltknopf kann der Bedienknopf 144 eingesetzt werden, wobei alternativ oder zusätzlich jedoch ein Ein- und Ausschalten des Diagnosesystems 114 oder des darin enthaltenen Computersystems 626 durch ein Zusammenfügen des Magazinmoduls 110 und des Diagnosemoduls 112 erfolgen kann.

Weiterhin weist das Diagnosemodul 112 ein integriertes Lanzettensystem (siehe z. B. Bezugsziffer 612 in Figur 6 unten) auf. Dieses Lanzettensystem 612, welches in Figur 1 aufgrund der perspektivischen Darstellung nicht abgebildet ist verfügt über eine Lanzettenkappe 146, welche eine in der Lanzettenkappe 146 aufgenommene Lanzettennadel (Bezugsziffer 628 in Figur 6 unten) bedeckt. Die Lanzettenkappe 146 weist an ihrer Vorderseite ein Lanzettenloch 630 (siehe ebenfalls Figur 6) auf, durch welches die Lanzettennadel 628 bei Betätigung des Lanzettensystems 612 kurzzeitig austreten kann, um eine Hautschicht zu perforieren und einen Blutstropfen zu erzeugen. Vorteilhafterweise genügen dabei Blutstropfen von weniger als einem Mikroliter. Die Lanzettenkappe 146 ist gleichzeitig als drehbares Stellsystem ausgeführt, wobei durch eine Drehung der Lanzettenkappe 146 eingestellt werden kann, wieweit die Lanzettennadel 628 bei Betätigung des Lanzettensystems 612 aus der Lanzettenkappe 146 bzw. dem Lanzettenloch 630, austritt. Auf diese Weise lässt sich insbesondere die Stichtiefe bei der Gewinnung von Blutstropfen einstellen, wodurch beispielsweise das Diagnosesystem 114 auf unterschiedliche Hautdicken angepasst werden kann oder wodurch das Volumen des zu erzeugenden Blutstropfens eingestellt wird. Das Lanzettensystem 612 ist über einen auf der der Vorderseite des Diagnosemoduls 112 gegenüber liegenden Stirnseite angeordneten Auslöseknopf 148 auslösbar.

Das in Figur 1 dargestellte Diagnosesystem 114 zeichnet sich dadurch aus, dass es zum einen eine günstige Griffposition durch Erfassen des Magazinmoduls 110 erlaubt. Gleichzeit können in dieser Griffposition vom Betrachter über das auf der Bedienoberfläche 140 angeordnete Display 142 leicht Informationen abgelesen werden und mit einer Hand das Diagnosesystem 114 bedient werden. Für den Transport des Systems lassen sich das Magazinmodul 110 und das Diagnosemodul 112 auf einfache Weise trennen, wobei beispielsweise automatisch das Diagnosemodul 112 abgeschaltet werden kann. Ein getrennter Transport der beiden Module 110 und 112 ist also möglich, so dass das Diagnosesystem 114 für den Transport Platz sparend zerlegt werden kann. Für eine Bedienung des Diagnosesystems 114 ist zunächst einmalig der Einsatz beider Hände erforderlich, wobei das Diagnosemodul 112 mit seiner diagnosemodulseitigen Schwalbenschwanzschiene 130 in die magazinseitige schwalbenschwanzförmige Nut 122 eingeschoben wird. Dabei wird beispielsweise das Diagnosesystem 114 automatisch gestartet, d.h. insbesondere beispielsweise ein in dem Diagnosesystem 114 enthaltenes Computersystem 626 initialisiert. Gleichzeitig wird durch das Zusammenfügen der beiden Module 110, 112 das in dem Diagnosemodul 112 enthaltene Lanzettensystem 612 gespannt Weiterhin wird bei dem Zusammenfügen der beiden Module 110, 112 ein elektrischer Kontakt zwischen dem Diagnosemodul 112 und dem Magazinmodul 110 hergestellt, wodurch einerseits im Magazinmodul 110 angeordnete Messelektroden (siehe z. B. Bezugsziffer 422 in Figur 4 unten) vom Diagnosemodul 112 ausgelesen werden können und wodurch andererseits über die ROM-Key-Kontakte 126, 138 Chargeninformationen über die in dem Magazinmodul 110 enthaltenen Teststreifen 310 automatisch an das Diagnosemodul 112 übertragen werden. Die Fehlerquelle einer benutzerseitigen falschen Eingabe der Chargeninformationen in das Diagnosesystem 114 bzw. die Fehlerquelle einer Störung einer drahtlosen Übertragung von Chargeninformationen wird dadurch vermieden (siehe oben).

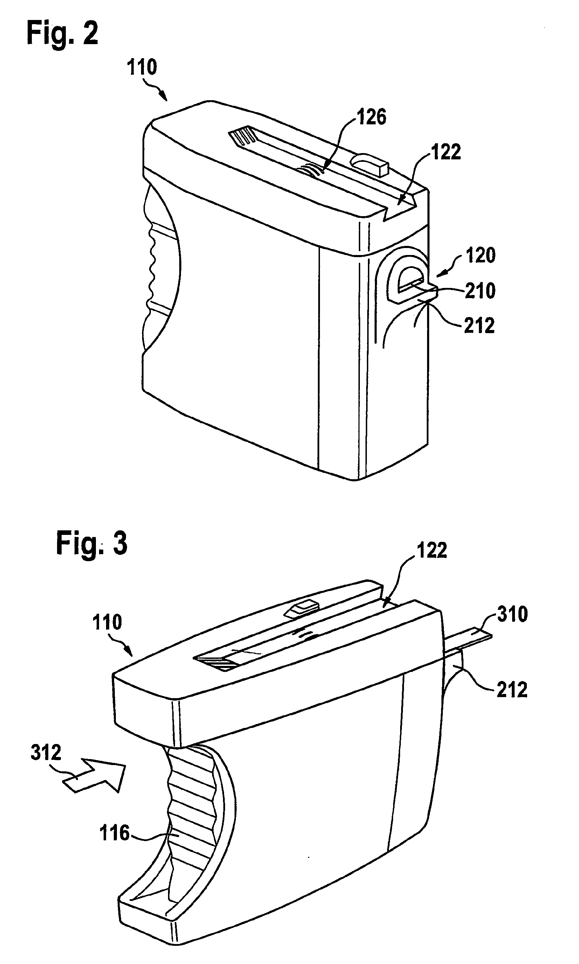

In den Figuren 2 und 3 ist das Magazinmodul 110 noch einmal in verschiedenen perspektivischen Ansichten dargestellt Dabei ist in Figur 2 eine Vorderansicht abgebildet, bei welcher die Ausgabeeinheit 120 im Detail dargestellt ist Ein wesentliches Element der Ausgabeeinheit 120 ist ein Ausgabeschlitz 210, welcher horizontal angeordnet ist und näherungsweise die Breite und die Dicke eines Teststreifens 310 aufweist Der Ausgabeschlitz 210 kann zusätzlich durch eine Dichtlippe (siehe z. B. Bezugsziffer 526 in Figur 4 unten), beispielsweise eine Gummidichtlippe, verschlossen sein, welche ein Ausschieben eines Teststreifens 310 aus dem Ausgabeschlitz 210 ermöglicht, jedoch ein Eindringen von Feuchtigkeit und Schmutz in den Innenraum des Magazinmoduls 110 weitgehend verhindert. Weiterhin weist die Ausgabeeinheit 120 eine Haltelippe 212 auf, welche einen Teststreifen 310 in Messposition (siehe Figur 3) unterstützt, so dass dieser teilweise in das Magazinmodul 110 hineinragt und dort von Messelektroden kontaktiert wird und teilweise aus dem Magazinmodul 110 herausragt, um dort beispielsweise einen Blutstropfen aufzunehmen.

In Figur 3 ist das Magazinmodul 110 in Messposition bei eingedrücktem Betätigungsknopf 116 dargestellt, wobei die Betätigungsrichtung des Betätigungsknopfs 116 durch den Pfeil 312 symbolisiert ist. Der Betätigungsknopf 116 hat in diesem Beispiel, wie unten näher erläutert, eine doppelte Funktion. Eine einmalige Betätigung des Betätigungsknopfs 116 in Betätigungsrichtung 312 bewirkt, dass ein Teststreifen 310 aus dem Magazinmodul 110 durch den Ausgabeschlitz 210 teilweise ausgeschoben wird und somit in einer Messposition (siehe oben) positioniert wird. In dieser Position können mit dem herausragenden Ende des Teststreifens 310 Blutstropfen aufgenommen werden, welche durch ein Kapillarsystem in den innerhalb des Magazinmoduls 110 liegenden Teil des Teststreifens 310 zu einem entsprechenden Elektrodensystem transportiert werden. Dieses Elektrodensystem, welches wie oben beschrieben, beispielsweise mit Enzymen und Mediatoren beschichtet ist, ist über Messelektroden (siehe Bezugsziffer 422 in Figur 4 unten) im Inneren des Magazinmoduls 110 kontaktiert, welche wiederum über Messelektroden-Leitungssystem (siehe Bezugsziffer 420 in Figur 4) und die Messelektrodenkontakte 124, 136 von Diagnosemodul 112 ausgelesen werden können. Eine erneute Betätigung des Betätigungsknopfs 116 in Betätigungsrichtung 312 nach erfolgter Messung bewirkt schließlich, dass der nunmehr verbrauchte Teststreifen 310 aus dem Magazinmodul 110 vollständig ausgeworfen wird, so dass er entsorgt werden kann.

In den Figuren 4 und 5 ist eine Schnittdarstellung des Magazinmoduls 110 abgebildet, wobei die Schnittdarstellung gemäß Figur 4 der perspektivischen Darstellung gemäß Figur 2 entspricht und die Schnittdarstellung gemäß Figur 5 der perspektivischen Darstellung gemäß Figur 3 mit in Betätigungsrichtung 312 eingedrücktem Betätigungsknopf 116. Das Magazinmodul 110 weist ein Gehäuse 410 auf, in welches ein Stapelmagazin 412 für Teststreifen 310 eingelassen ist. In diesem Stapelmagazin 412 liegen die Teststreifen 310 horizontal ausgerichtet übereinander und werden durch ein Federsystem 414 nach oben gedrückt. Im Stapelmagazin 412 sind die Teststreifen 310 zusätzlich von einem Trockenmittel 416 umgeben, beispielsweise Silicagel, welches die Luftfeuchtigkeit innerhalb des Stapelmagazins 412 reduziert.

Weiterhin ist in den Abbildungen gemäß Figuren 4 und 5 wiederum die schwalbenschwanzförmige Nut 122 und die Mitnehmernase 128 zu erkennen. Auch die Anordnung der magazinseitigen Messelektrodenkontakte 124 und der magazinseitigen ROM-Key-Kontakte 126 ist zu erkennen. Die ROM-Key-Kontakte 126 stehen in direkter Verbindung mit einem Datenspeicher 418 (ROM-Key), welcher Chargeninformationen über die Teststreifen 310 enthält Diese Chargeninformationen können über die ROM-Key-Kontakte 126 vom Diagnosemodul 112 ausgelesen werden. Die magazinseitigen Messelektrodenkontakte 124 stehen über ein Messelektroden-Leitungssystem 420 in Verbindung mit Messelektroden 422, welche oberhalb des Stapelmagazins 412 angeordnet sind. Befindet sich ein Teststreifen 310 in Messposition (siehe Figur 5 oberster Teststreifen 310), so kontaktieren, wie oben beschrieben, die Messelektroden 422 die auf dem Teststreifen 310 befindlichen Elektroden, so dass diese Elektroden auf dem Teststreifen 310 über die magazinseitigen Messelektrodenkontakte 124 und die diagnosemodulseitigen Messelektrodenkontakte 136 vom Diagnosemodul 112 ausgelesen und dort in einem Computersystem 626 ausgewertet werden können.

Weiterhin weist das Magazinmodul 110 eine Betätigungsvorrichtung 510 auf, deren Wirkungsweise anhand eines Vergleichs der Figuren 4 und 5 beschrieben werden soll. Die Betätigungsvorrichtung 510 weist, wie oben bereits beschrieben, einen Betätigungsknopf 116 auf, welcher beispielsweise mit dem Daumen einer Hand oder mit dem Handballen in Betätigungsrichtung 312 (siehe Figur 3) eingedrückt werden kann. Weiterhin weist die Betätigungsvorrichtung 510 einen Federmechanismus 512 auf, welcher eine Kraft auf den Betätigungsknopf 116 entgegen der Betätigungsrichtung 312 (siehe Figur 3) ausübt. Der Federmechanismus 512 ist in diesem Ausführungsbeispiel aus flexiblem Material, beispielsweise einem Kunststoff, hergestellt und weist beispielsweise die Form des griechischen Buchstabens Omega auf. Weiterhin weist die Betätigungsvorrichtung 510 eine über eine Achse 514 drehbar am Betätigungsknopf 116 gelagerte Schubstange 516 auf, welche an ihrem der Achse 514 gegenüber liegenden Ende mit einem Schubstück 518 verbunden ist. Die Schubstange 516 ragt dabei in das Stapelmagazin 412 hinein, so dass die Achse 514 außerhalb des Stapelmagazins und das Schubstück 518 innerhalb des Stapelmagazins 412 angeordnet sind. Der Eintritt der Schubstange 516 in das Stapelmagazins 412 ist dabei durch eine erste Dichtlippe 520 abgedichtet, um das Eindringen von Feuchtigkeit in das Stapelmagazin 412 zu verringern bzw. zu vermindern.

Das Schubstück 518, welches an der Achse 514 gegenüber liegenden Ende der Schubstange 516 leicht um die Schubstange 516 drehbar gelagert ist, weist zwei Schubkanten 522, 524 auf. Eine erste Schubkante 522, auch Messkante genannt, welche näher zur Achse 514 angeordnet ist, und eine zweite Schubkante 524, auch Auswurfkante genannt, welche weiter von der Achse 514 beabstandet angeordnet ist Die Funktion dieser beiden Schubkanten 522, 524 wird anhand eines Vergleichs der Figuren 4 und 5 deutlich. Bei einer ersten Betätigung des Betätigungsknopfs 116 wird der Federmechanismus 512 zusammengedrückt und die Schubstange 516 durch die erste Dichtlippe 520 hindurch weiter in das Stapelmagazin 412 hineingeschoben, wobei der oberste Teststreifen 310 an seinem zur Achse 514 hin gelegenen Ende von der ersten Schubkante 522 erfasst und durch den mit einer zweiten Dichtlippe 526 versehenen Ausgabeschlitz 210 teilweise ausgeschoben wird. Bei vollständig eingedrücktem Betätigungsknopf 116, wie in Figur 5 dargestellt, befindet sich der Teststreifen 310 in einer Messposition. Hierbei kann, wie oben beschrieben, über die Spitze 528 des Teststreifens 310 ein Blutstropfen aufgenommen werden, welcher dann über ein Kapillarsystem im Inneren des Teststreifens 310 zu einem in der in Figur 5 dargestellten Messposition unterhalb der Messelektroden 422 angeordnetem Elektrodensystem, welches durch die Messelektroden 422 kontaktiert wird, befördert wird (siehe oben).

Wird anschließend keine Kraft mehr auf den Betätigungsknopf 116 ausgeübt, so bewegt sich der Betätigungsknopf 116, getrieben durch die Kraft durch den Federmechanismus 512, wieder in seine in Figur 4 dargestellte Ausgangsposition zurück. Dabei wird auch die Schubstange 516 und somit auch das Schubstück 518 wieder in die in Figur 4 dargestellte Position bewegt. Der oberste Teststreifen 310 verbleibt jedoch in der in der Figur 5 dargestellten Messposition. Dabei wirken die Messelektroden 422 als Gegenstück zu dem Federsystem 414 und verhindern, dass weitere Teststreifen 310 sich von unten nach oben bewegen können. Wird anschließend der Betätigungsknopf 116 erneut in Betätigungsrichtung 312 bewegt, so wird die Schubstange 516 mit dem daran drehbar gelagerten Schubstück 518 erneut in das Stapelmagazin 412 eingeschoben. Das Schubstück 518 ist dabei vorzugsweise derart drehbar um die Schubstange 516 gelagert, dass ein leichtes Übergewicht auf Seiten der zweiten Schubkante 524 aufweist Demzufolge erfasst bei diesem zweiten Einschieben der Schubstange 516 in das Stapelmagazin 412 die zweite Schubkante 524, das der Achse 514 näher gelegene Ende des obersten Teststreifens 310. Da diese zweite Schubkante 524 weiter von der Achse 514 beabstandet ist als die erste Schubkante 522, wird bei diesem zweiten Einschieben des Schubstücks 518 der Teststreifen 310 weiter aus dem Ausgabeschlitz 210 ausgeschoben, so dass er den Ausgabeschlitz 210 schließlich ganz verlässt oder soweit ausgeschoben wird, dass er aufgrund des nunmehr außerhalb des Magazinmoduls 110 angeordneten Schwergewichts durch seine Schwerkraft vollständig aus dem Ausgabeschlitz 210 ausgeworfen wird. Alternativ oder zusätzlich kann auch das Schubstück 518 beim Einschieben in das Stapelmagazin 412 die Messelektroden 422 nach oben schieben, so dass das Schubstück 518 näher an den Ausgabeschlitz 210 herangeführt werden kann. Insgesamt ergibt also der dargestellte Mechanismus ein System, bei dem bei einer erstmaligen Betätigung der Betätigungsvorrichtung 510 zunächst ein Teststreifen 310 in eine Messposition geschoben wird und anschließend bei einer zweiten Betätigung der Betätigungsvorrichtung 510 derselbe Teststreifen 310, der nunmehr "verbraucht" ist, ausgeworfen wird.

In den Figuren 6 und 7 ist eine Schnittdarstellung eines Diagnosemoduls 112 stark vereinfacht dargestellt. Dabei wird insbesondere deutlich, wie eine in dem Diagnosemodul 112 angeordnete Spannvorrichtung 610 zum Spannen eines Lanzettensystems 612 beim Verbinden eines Magazinmoduls 110 mit dem Diagnosemodul 112 gespannt wird. Die Spannvorrichtung 610 weist eine Spiralfeder 614 auf, sowie einen Spannschlitten 616. Der Spannschlitten 616 ist noch einmal in Figur 8 in perspektivischer Darstellung abgebildet. Wird das Magazinmodul 110 mittels der oben beschriebenen, die schwalbenschwanzförmige Nut 122 am Magazinmodul 110 und die diagnosemodulseitige Schwalbenschwanzschiene 130 aufweisende Einschubverbindung in Einschubrichtung 618 mit dem Diagnosemodul 112 verbunden, so ragt die Mitnehmernase 128 auf der Oberseite des Magazinmoduls 110 durch den Mitnehmerschlitz 134 in der Montageplatte 132 in das Diagnosemodul 112 hinein und kann dort eine ebenfalls in den Mitnehmerschlitz 134 hineinragende Spannnase 620 des Spannschlittens 616 ergreifen. Dabei wird der Spannschlitten 616 in Einschubrichtung 618 geschoben. Der Spannschlitten 616 ist über ein Federlager 622 mit der Spiralfeder 614 des Lanzettensystems 612 verbunden. Bewegt sich der Spannschlitten 616 in Einschubrichtung 618, so wird die Spiralfeder 614 des Lanzettensystems 612 ebenfalls in Einschubrichtung 618 zusammengestaucht. Dadurch wird das Lanzettensystem 612 gespannt (siehe Figur 7, gespannte Position). Bei vollständig miteinander verbundenem Magazinmodul 110 und Diagnosemodul 112 kommen auch, wie in Figur 7 ersichtlich, die magazinseitigen Messelektrodenkontakte 124 mit den diagnosemodulseitigen Messelektrodenkontakten 136 in Kontakt, und die magazinseitigen ROM-Key-Kontakte 126 kommen mit den diagnosemodulseitigen ROM-Key-Kontakten 138 in Kontakt. Somit können diese Kontakte 124, 136, 126, 138 über Messleitungen 624 von einem in dem Diagnosemodul 112 angeordneten Computersystem 626 ausgelesen werden. Auf diese Weise stehen dem Computersystem 626 sowohl die genannten Chargeninformationen über die Teststreifen 310 als auch die eigentlichen, von den Messelektroden 422 gelieferten Signale der Blutglukosekonzentrationsmessung.

Das Lanzettensystem 612 verfügt über eine auswechselbare Lanzettennadel 628. Wird in dem in Figur 7 dargestellten gespannten Zustand des Lanzettensystems 612 der (hier nicht dargestellte) Auslöseknopf 148 (siehe Figur 1) betätigt, so wird die bis dahin blockierte Lanzettennadel 628 freigegeben (beispielsweise durch Freigeben eines entsprechenden Sperrhakens), und die gespannte Spiralfeder 614 wirkt auf die Lanzettennadel 628 ein und treibt diese durch ein Lanzettenloch 630 in der Lanzettenkappe 146 schlagartig aus. Diese Stechbewegung der Lanzettennadel 628 wird gebremst durch einen Anschlag 632, welcher an den Rändern des Lanzettenlochs 630 anschlägt und somit eine maximale Austrittstiefe der Lanzettennadel 628 aus dem Lanzettenloch 630 definiert. Durch Verändern der Lage der Lanzettenkappe 146 relativ zum restlichen Diagnosemodul 112 (beispielsweise Mittels einer Drehbewegung und eines Gewindes, über welches die Lanzettenkappe 146 mit dem restlichen Diagnosemodul 112 verbunden ist) kann somit eine Stechtiefe des Lanzettensystems 612 eingestellt werden.

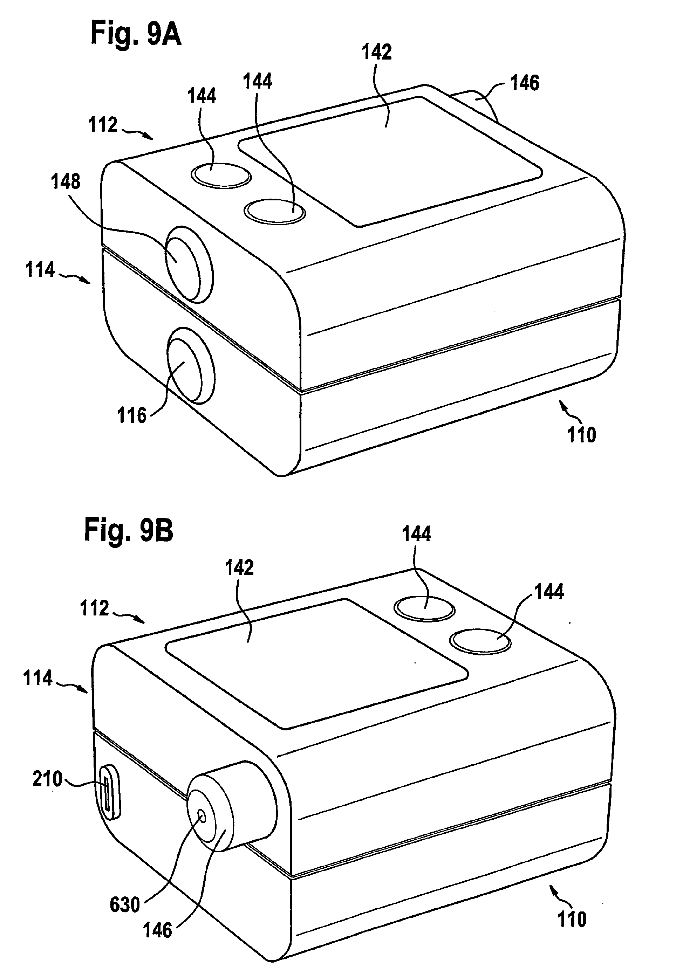

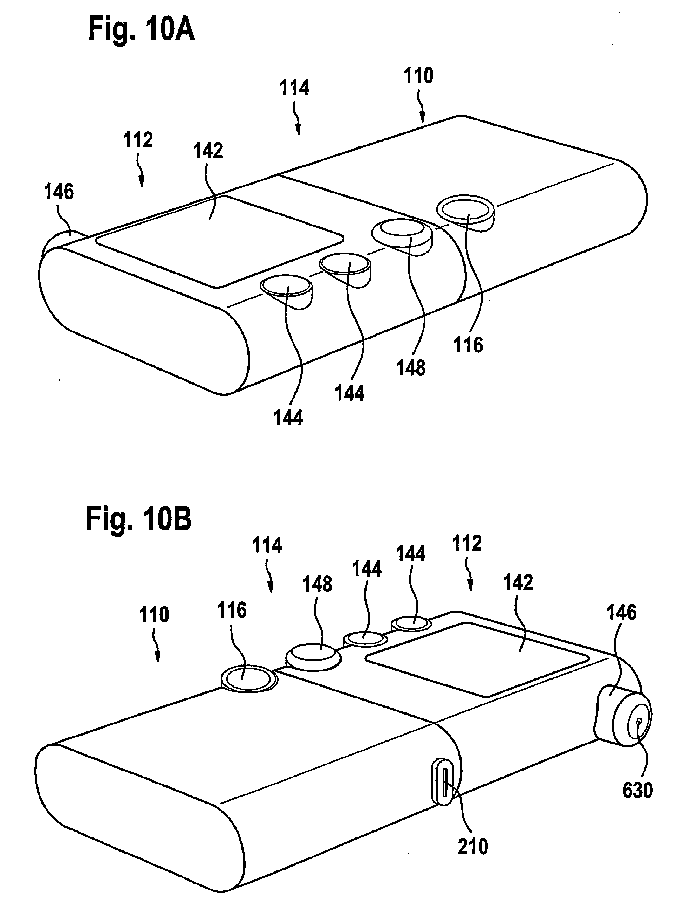

In den Figuren 9A und 9B sowie in den Figuren 10A und 10B sind in perspektivischer Darstellung zwei weitere, zu der in den Figuren 1 bis 8 dargestellten ersten Ausführungsform alternative erfindungsgemäße Ausführungsformen eines Diagnosesystems 114 dargestellt. Dabei zeigen jeweils die Figuren 9A und 10A eine Darstellung in Rückansicht, die Figuren 9B und 10B eine Darstellung in Vorderansicht, also von der Seite, auf der die Lanzettenkappe 146 angeordnet ist.

Die Funktionalität der Elemente, insbesondere der Bedienelemente 144, des Betätigungsknopfes 116 und des Auslöseknopfes 148 ist identisch bzw. funktionsgleich zum oben dargestellten ersten Ausführungsbeispiel. Auch der Aufbau des Diagnosemoduls 112 und des Magazinmoduls 110 kann prinzipiell gleich oder ähnlich dem oben beschrieben Aufbau erfolgen.

Der wesentliche Unterschied zwischen den Ausführungsbeispielen gemäß den Figuren 9A, 9B, 10A und 10B zum oben genannten ersten Ausführungsbeispiel besteht jedoch in der Geometrie der Anordnung von Magazinmodul 110 und Diagnosemodul 112 relativ zueinander und im Zusammenbau zum fertigen Diagnosesystem 114. Während das oben dargestellte, bevorzugte erste Ausführungsbeispiel im Wesentlichen die Form eines "T"s (auch "Pistolen"-Anordnung genannt) aufweist, weist das in den Figuren 9A und 9B dargestellte zweite Ausführungsbeispiel im zusammengesetzten Zustand des Diagnosesystems 114 eine "Stapelform" auf mit einer Geometrie, die im Wesentlichen einem Quader mit einer quadratischen Grundfläche von ca. 50 mm x 50 mm und einer Höhe von ca. 30 mm entspricht. Näherungsweise die Hälfte dieses Quaders wird dabei durch das Magazinmodul 110 gebildet (welches also die Form eines rechteckigen Quaders mit einer Höhe von ca. 15 mm aufweist) und die andere Hälfte durch das Diagnosemodul 112. Die Stapelform gemäß Figur 9A und 9B ist auch im zusammengesetzten Zustand des Diagnosesystems 114 besonders platzsparend. Auch andere Abmessungen als die genannten Abmessungen sind möglich, insbesondere auch ungleiche Abmessungen von Magazinmodul 110 und Diagnosemodul 112.

Entsprechend ist in den Figuren 10A und 10B eine dritte alternative Ausgestaltung dargestellt. Hier weisen Magazinmodul 110 und Diagnosemodul 112, analog zu den Figuren 9A und 9B, wiederum Quaderform (Kantenlängen wiederum 50 mm x 50 mm x 15 mm) auf. Beiden Module 110, 112 sind jedoch in diesem Ausführungsbeispiel an ihren Stirnseiten zusammengefügt, so dass eine "Reihenform" entsteht, also ein Quader mit einer Kantenlänge von ca. 100 mm x 50 mm x 15 mm. Diese Form ist besonders griffgünstig, wobei gleichzeitig jedoch gute Ablesbarkeit und auch im zusammengesetzten Zustand geringer Platzbedarf gewährt bleiben. Auch andere Abmessungen als die genannten Abmessungen sind möglich, insbesondere auch ungleiche Abmessungen von Magazinmodul 110 und Diagnosemodul 112.

Beide Module 110 und 112 können auch in den Ausführungsbeispielen gemäß den Figuren 9A, 9B, 10A, 10B, analog zu der in Figur 1 dargestellten Verbindung, beispielsweise über eine schwalbenschwanzförmige Nut 122 und eine diagnosemodulseitige Schwalbenschwanzschiene 130 (wobei natürlich Nut 122 und Schiene 130 auch vertauscht sein können) miteinander verbunden sein. Auch das Spannen des Lanzettensystems 612 und der Austausch von Daten und Signalen kann analog zum Ausführungsbeispiel gemäß Figur 1 erfolgen.

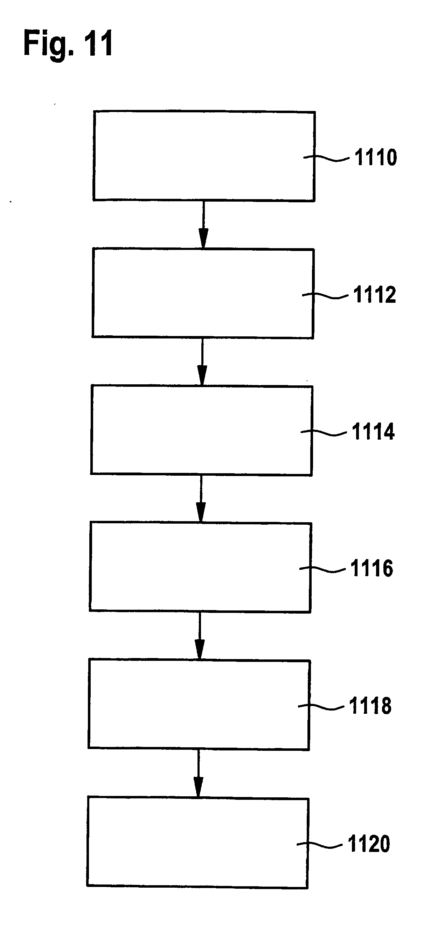

In Figur 11 ist schließlich ein schematischer Ablaufplan einer möglichen Ausführung eines erfindungsgemäßen Verfahrens zur Bedienung eines erfindungsgemäßen Diagnosesystems 114 dargestellt. Die dargestellten Verfahrensschritte müssen nicht notwendigerweise in der abgebildeten Reihenfolge durchgeführt werden. Auch können weitere, nicht dargestellte Verfahrensschritte durchgeführt werden.

Zunächst wird in Verfahrensschritt 1110 ein Magazinmodul 110 mit Teststreifen 310, beispielsweise gemäß dem Ausführungsbeispiel gemäß Figur 1, mit einem Diagnosemodul 112 verbunden, wobei ein Lanzettensystem 612 gespannt wird. Anschließend wird in Verfahrensschritt 1112 das Lanzettensystem 612 ausgelöst, beispielsweise durch Betätigen eines Auslöseknopfes 148, wodurch eine flüssige Probenmenge, insbesondere ein Blutstropfen, vorbereitet wird.

In Verfahrensschritt 1114 wird dann, beispielsweise durch Betätigen einer Betätigungsvorrichtung 510 gemäß den Figuren 4 und 5, ein Teststreifen 310 aus dem Magazinmodul 110 in eine Messposition befördert. Anschließend wird in Verfahrensschritt 1116 die Probenmenge mit dem Teststreifen 310 in Verbindung gebracht. Dann wird in Schritt 1118, beispielsweise anhand des oben beschriebenen Verfahrens einer Strom-Spannungs-Messung, die Stoffkonzentration mindestens eines vorgegebenen Stoffes, beispielsweise Glukose, in der Probenmenge ermittelt. Anschließend kann in dem optionalen Verfahrensschritt 1120 der gerade benutzte Teststreifen 310 ausgeworfen werden, beispielsweise, wie oben beschrieben, mittels einer erneuten Betätigung der Betätigungsvorrichtung 510.
- 110: Magazinmodul
- 112: Diagnosemodul
- 114: Diagnosesystem
- 116: Betätigungsknopf
- 118: Ausbuchtung in Stirnseite des Magazinmoduls 110
- 120: Ausgabeeinheit
- 122: schwalbenschwanzförmige Nut
- 124: magazinseitige Messelektrodenkontakte
- 126: magazinseitige ROM-Key-Kontakte
- 128: Mitnehmernase
- 130: diagnosemodulseitige Schwalbenschwanzschiene
- 132: Montageplatte
- 134: Mitnehmerschlitz
- 136: diagnosenmodulseitige Messelektrodenkontakte
- 138: diagnosemodulseitige ROM-Key-Kontakte
- 140: Bedienoberfläche
- 142: Display
- 144: Bedienknopf
- 146: Lanzettenkappe
- 148: Auslöseknopf

- 210: Ausgabeschlitz
- 212: Haltelippe

- 310: Teststreifen
- 312: Betätigungsrichtung des Betätigungsknopfes 116

- 410: Gehäuse
- 412: Stapelmagazin
- 414: Federsystem
- 416: Trockenmittel
- 418: Datenspeicher (ROM-Key)
- 420: Messelektroden-Leitungssystem
- 422: Messelektroden

- 510: Betätigungsvorrichtung
- 512: Federmechanismus
- 514: Achse
- 516: Schubstange
- 518: Schubstück
- 520: erste Dichtlippe
- 522: erste Schubkante, Messkante
- 524: zweite Schubkante, Auswurfkante
- 526: zweite Dichtlippe
- 528: Spitze des Teststreifens 310 in Messposition

- 610: Spannvorrichtung
- 612: Lanzettensystem
- 614: Spiralfeder
- 616: Spannschlitten
- 618: Einschubrichtung
- 620: Spannnase
- 622: Federlager
- 624: Messleitungen
- 626: Computersystem
- 628: Lanzettennadel
- 630: Lanzettenloch
- 632: Anschlag

- 1110: Verbindung von Magazinmodul 110 und Diagnosemodul 112
- 1112: Auslösung des Lanzettensystems 612
- 1114: Beförderung eines Teststreifens 310 in eine Messposition
- 1116: Inverbindungbringen einer Probenmenge mit dem Teststreifen 310
- 1118: Ermittlung einer Stoffkonzentration
- 1120: Auswerfen des Teststreifens 310

## Patentansprüche

1. Diagnosesystem (114) zum Ermitteln von Stoffkonzentrationen in flüssigen Proben, insbesondere zur Blutzuckermessung im menschlichen Blut, mit
a) einem Diagnosemodul (112) mit einem in das Diagnosemodul (112) integriertem Lanzettensystem (612); und
b) einem vom Diagnosemodul (112) getrennten Magazinmodul (110) zum Speichern von Teststreifen (310),
**gekennzeichnet durch**
- eine Verbindungsvorrichtung (122, 130) zur Verbindung des Magazinmoduls (110) mit dem Diagnosemodul (112),
- wobei die Verbindungsvorrichtung (122, 130) eine Spannvorrichtung (610) zum Spannen des Lanzettensystems (612) aufweist und/oder mit einer Spannvorrichtung (610) zum Spannen des Lanzettensystems (612) derart verbunden ist, dass bei der Verbindung des Magazinmoduls (110) mit dem Diagnosemodul (112) das Lanzettensystem (612) gespannt wird.

2. Diagnosesystem (114) gemäß dem vorhergehenden Anspruch, **gekennzeichnet durch** eine Datenaustauschvorrichtung (126, 138, 418) zum Austauschen von Chargeninformadonen der Teststreifen (310) zwischen dem Magazinmodul (110) und dem Diagnosemodul (112).

3. Diagnosesystem (114) gemäß dem vorhergehenden Anspruch, **dadurch gekennzeichnet, dass** die Datenaustauschvorrichtung (126, 138, 418) einen im Magazinmodul (110) angeordneten Transponder und ein im Diagnosemodul (112) angeordnetes Transponderlesegerät (626) aufweist.

4. Diagnosesystem (114) gemäß einem der beiden vorhergehenden Ansprüche, **dadurch gekennzeichnet, dass** die Datenaustauschvorrichtung (126, 138, 418) einen im Magazinmodul (110) angeordneten Datenspeicher (418), insbesondere ein EPROM aufweist.

5. Diagnosesystem (114) gemäß einem der drei vorhergehenden Ansprüche, **dadurch gekennzeichnet, dass** die Datenaustauschvorrichtung (126, 138, 418) mindestens einen in der Verbindungsvorrichtung (122, 130) angeordneten elektrischen Kontakt (126, 138) aufweist.

6. Diagnosesystem (114) gemäß einem der vorhergehenden Ansprüche, **dadurch gekennzeichnet, dass** die Verbindungsvorrichtung (122, 130) eine Einschubverbindung (122, 130) aufweist.

7. Diagnosesystem (114) gemäß dem vorhergehenden Anspruch, **dadurch gekennzeichnet, dass** die Einschubverbindung (122, 130) eine Schwalbenschwanzführung (122, 130) aufweist.

8. Diagnosesystem (114) gemäß einem der vorhergehenden Ansprüche, **dadurch gekennzeichnet, dass** die Spannvorrichtung (610) einen mit einem Federsystem (614) verbundenen Spannschlitten (616) aufweist.

9. Diagnosesystem (114) gemäß einem der vorhergehenden Ansprüche, **dadurch gekennzeichnet, dass** das Diagnosemodul (112) ein Display (142) und/oder ein Computersystem (626) aufweist.

10. Diagnosesystem (114) gemäß einem der vorhergehenden Ansprüche, **dadurch gekennzeichnet, dass** das Diagnosesystem (114) die folgenden Bedienelemente (510, 148, 144) aufweist: eine Betätigungsvorrichtung (510) zum Bedienen des Magazinmoduls (110), eine Auslösevorrichtung (148) zum Auslösen des Lanzettensystems (612) und ein Bediensystem (144) zum Bedienen des Diagnosemoduls (112).

11. Diagnosesystem (114) gemäß dem vorhergehenden Anspruch, **gekennzeichnet durch** ein im Wesentlichen vertikales Griffstück (110) und eine im Wesentlichen horizontale Bedienoberfläche (140).

12. Diagnosesystem (114) gemäß dem vorhergehenden Anspruch, **dadurch gekennzeichnet, dass** die Bedienoberfläche (140) mindestens ein Bedienelement (144) aufweist.

13. Diagnosesystem (114) gemäß einem der beiden vorhergehenden Ansprüche, **dadurch gekennzeichnet, dass** mindestens drei Bedienelemente (510, 148, 144) bei mit dem Diagnosemodul (112) verbundenem Magazinmodul (110) mit einer Hand bedienbar sind.

14. Diagnosesystem (114) gemäß einem der vorhergehenden Ansprüche, **dadurch gekennzeichnet, dass** das Magazinmodul (110) mindestens eine Messelektrode (422) aufweist, wobei bei mit dem Diagnosemodul (112) verbundenem Magazinmodul (110) das Diagnosemodul (112) über die mindestens eine Messelektrode (422) mit mindestens einem Teststreifen (310) verbindbar ist.

15. Diagnosesystem (114) gemäß einem der vorhergehenden Ansprüche, **dadurch gekennzeichnet, dass** das Magazinmodul (110) ein Stapelmagazin (412) für Teststreifen (310) aufweist.

16. Diagnosesystem (114) gemäß einem der vorhergehenden Ansprüche, **dadurch gekennzeichnet, dass** das Magazinmodul (110) eine Betätigungsvorrichtung (510) zum Ausgeben von Teststreifen (310) aufweist.

17. Diagnosesystem (114) gemäß dem vorhergehenden Anspruch, **dadurch gekennzeichnet, dass** die Betätigungsvorrichtung (510) folgendes aufweist: einen Betätigungsknopf (116), eine Schubstange (516) und ein Schubstück (518) zum Verschieben eines Teststreifens (310), wobei das Schubstück (518) mindestens eine erste Schubkante (522) und eine zweite Schubkante (524) zum Erfassen eines Teststreifens (310) aufweist.

18. Diagnosesystem (114) gemäß einem der vorhergehenden Ansprüche, **gekennzeichnet durch** ein in das Magazinmodul (110) eingebrachtes Trockenmittel (416) zur Verringerung der Luftfeuchtigkeit.

19. Diagnosystem (114) nach einem der Ansprüch 1-18, wobei die Verbindungsvorrichtung (122, 130) eine Betätigungevorrichtung (510) zum Bedienen des Magazinmoduls (110), insbesondere zur Ansgebe mindestens eines Teststreifens (310), auf weist und/oder mit einer Betätigungsvorrichtung (510) zum Bedienen des Magazin moduls (110), insbesondere zur Ausgebe mindestens eines Teststreifens (310), verbunden ist, dergestals, dass bei der Verbindung des Magazin moduls (110) mit dem Diagnosemodul (112) mindestens eine Teststreiten (310) ausgegeben wird.

20. Verfahren zum Bedienen eines Diagnosesystems (114) gemäß einem der vorhergehenden. Ansprüche, mit folgenden Schritten:
a) ein Magazinmodul (110) mit Teststreifen (310) wird mit einem Diagnosemodul (112) verbunden, wobei ein Lanzettensystem (612) gespannt wird und/oder ein Teststreifen (310) ausgegeben wird;
b) das Lanzettensystem (612) wird ausgelöst, wodurch eine flüssige Probenmenge, insbesondere ein Blutstropfen, vorbereitet wird;
c) ein Teststreifen (310) aus dem Magazinmodul (110) wird in eine Messposition befördert;
d) die Probenmenge wird mit dem Teststreifen (310) in Verbindung gebracht; und
e) eine Stoffkonzentration mindestens eines vorgegebenen Stoffes in der Probenmenge wird ermittelt.

21. Verfahren gemäß dem vorhergehenden Anspruch, **dadurch gekennzeichnet, dass** in Verfahrensschritt a) ein Computersystem (626) gestartet wird.

22. Verfahren gemäß einem der beiden vorhergehenden Ansprüche, **dadurch gekennzeichnet, dass** in Verfahrenschritt a) Chargeninformationen der Teststreifen (310) zwischen dem Magazinmodul (110) und dem Diagnosemodul (112) ausgetauscht werden.

23. Verfahren gemäß dem vorhergehenden Anspruch, **dadurch gekennzeichnet, dass** bei Durchführung von Verfahrensschritt e) die Chargeninformationen der Teststreifen (310) berücksichtigt werden.

24. Verfahren gemäß einem der vorhergehenden Verfahrensansprüche, **gekennzeichnet, durch** folgenden zusätzlichen Schritt:
f) der Teststreifen (310) wird ausgeworfen.

25. Verfahren gemäß einem der vorhergehenden Verfahrensansprüche, **dadurch gekennzeichnet, dass** in den Verfahrensschritten c) und f) dieselbe Betätigungsvorrichtung (510) betätigt wird.

26. Verfahren gemäß einem der vorhergehenden Verfahrensansprüche, **dadurch gekennzeichnet, dass** in Verfahrensschritt e) ein Strom als Funktion einer Spannung zwischen mindestens zwei mit dem Teststreifen (310) verbundenen Elektroden gemessen wird.

27. Verfahren gemäß einem der vorhergehenden Verfahrensansprüche, **dadurch gekennzeichnet, dass** in Verfahrensschritt b) vor dem Auslösen des Lanzettensystems (612) die gewünschte flüssige Probenmenge am Diagnosesystem (114) eingestellt wird.

## Claims

1. Diagnostic system (114) for determining substance concentrations in liquid samples, in particular for measuring blood glucose in human blood, with
a) a diagnostic module (112) with a lancet system (612) integrated into the diagnostic module (112); and
b) a magazine module (110) for storing test strips (310) which is separate from the diagnostic module (112),
**characterized by**
- a connecting device (122, 130) for connecting the magazine module (110) to the diagnostic module (112),
- the connecting device (122, 130) having a tensioning device (610) for tensioning the lancet system (612) and/or being connected to a tensioning device (610) for tensioning the lancet system (612) in such a way that, upon connection of the magazine module (110) to the diagnostic module (112), the lancet system (612) is tensioned.

2. Diagnostic system (114) according to the preceding claim, **characterized by** a data exchange device (126, 138, 418) for exchange of batch information on the test strips (310) between the magazine module (110) and the diagnostic module (112).

3. Diagnostic system (114) according to the preceding claim, **characterized in that** the data exchange device (126, 138, 418) has a transponder arranged in the magazine module (110), and a transponder reading unit (626) arranged in the diagnostic module (112).

4. Diagnostic system (114) according to one of the preceding two claims, **characterized in that** the data exchange device (126, 138, 418) has a data memory (418), in particular an EPROM, arranged in the magazine module (110).

5. Diagnostic system (114) according to one of the preceding three claims, **characterized in that** the data exchange device (126, 138, 418) has at least one electrical contact (126, 138) arranged in the connecting device (122, 130).

6. Diagnostic system (114) according to one of the preceding claims, **characterized in that** the connecting device (122, 130) has a push-in connection (122, 130).

7. Diagnostic system (114) according to the preceding claim, **characterized in that** the push-in connection (122, 130) has a dovetail guide (122, 130).

8. Diagnostic system (114) according to one of the preceding claims, **characterized in that** the tensioning device (610) has a tensioning slide (616) connected to a spring system (614).

9. Diagnostic system (114) according to one of the preceding claims, **characterized in that** the diagnostic module (112) has a display (142) and/or a computer system (626).

10. Diagnostic system (114) according to one of the preceding claims, **characterized in that** the diagnostic system (114) has the following operating elements (510, 148, 144): an actuating device (510) for operating the magazine module (110), a trigger device (148) for triggering the lancet system (612), and an operating system (144) for operating the diagnostic module (112).

11. Diagnostic system (114) according to the preceding claim, **characterized by** a substantially vertical grip portion (110) and a substantially horizontal operating surface (140).

12. Diagnostic system (114) according to the preceding claim, **characterized in that** the operating surface (140) has at least one operating element (144).

13. Diagnostic system (114) according to one of the preceding two claims, **characterized in that** at least three operating elements (510, 148, 144) can be operated by one hand when the magazine module (110) is connected to the diagnostic module (112).

14. Diagnostic system (114) according to one of the preceding claims, **characterized in that** the magazine module (110) has at least one measurement electrode (422) and, when the magazine module (110) is connected to the diagnostic module (112), the diagnostic module (112) is connectable to at least one test strip (310) via the at least one measurement electrode (422).

15. Diagnostic system (114) according to one of the preceding claims, **characterized in that** the magazine module (110) has a stack magazine (412) for test strips (310).

16. Diagnostic system (114) according to one of the preceding claims, **characterized in that** the magazine module (110) has an actuating device (510) for output of test strips (310).

17. Diagnostic system (114) according to the preceding claim, **characterized in that** the actuating device (510) has the following: an actuating button (116), a push rod (516) and a push element (518) for moving a test strip (310), the push element (518) having at least a first pushing edge (522) and a second pushing edge (524) for holding a test strip (310).

18. Diagnostic system (114) according to one of the preceding claims, **characterized by** a desiccant (416) introduced into the magazine module (110) in order to reduce the air moisture.

19. Diagnostic system (114) according to one of Claims 1-18, wherein the connecting device (122, 130) has an actuating device (510) for operating the magazine module (110), in particular for output of at least one test strip (310), and/or is connected to an actuating device (510) for operating the magazine module (110), in particular for outputting at least one test strip (310), in such a way that, upon connection of the magazine module (110) to the diagnostic module (112), at least one test strip (310) is output.

20. Method for operating a diagnostic system (114) according to one of the preceding claims, said method having the following steps:
a) a magazine module (110) with test strips (310) is connected to a diagnostic module (112), with a lancet system (612) being tensioned and/or a test strip (310) being output;
b) the lancet system (612) is triggered, as a result of which a quantity of liquid sample, in particular a drop of blood, is made ready;
c) a test strip (310) is conveyed from the magazine module (110) to a measurement position;
d) the sample quantity is brought into contact with the test strip (310); and
e) a substance concentration, at least of a predefined substance, in the sample quantity is determined.

21. Method according to the preceding claim, **characterized in that**, in method step a), a computer system (626) is started up.

22. Method according to one of the preceding two claims, **characterized in that**, in method step a), batch information on the test strips (310) is exchanged between the magazine module (110) and the diagnostic module (112).

23. Method according to the preceding claim, **characterized in that** the batch information on the test strips (310) is taken into account when carrying out method step e).

24. Method according to one of the preceding method claims, **characterized by** the following additional step:
f) the test strip (310) is ejected.

25. Method according to one of the preceding method claims, **characterized in that**, in method steps c) and f), the same actuating device (510) is actuated.

26. Method according to one of the preceding method claims, **characterized in that**, in method step e), a current is measured as a function of a voltage between at least two electrodes connected to the test strip (310).

27. Method according to one of the preceding method claims, **characterized in that**, in method step b), before triggering of the lancet system (612), the desired quantity of liquid sample is set on the diagnostic system (114).

## Revendications

1. Système de diagnostic (114) destiné à déterminer la concentration de substances présentes dans des échantillons liquides, et en particulier pour mesurer le sucre présent dans le sang humain, le système présentant :
a) un module de diagnostic (112) doté d'un système de lancette (612) intégré dans le module de diagnostic (112) et
b) un module de magasin (110) pour conserver des rubans de tests (310) qui est séparé du module de diagnostic (112),
**caractérisé par**
- un dispositif de liaison (122, 130) pour relier le module de magasin (110) au module de diagnostic (112),
- le dispositif de liaison (122, 130) présentant un dispositif de serrage (610) pour serrer le système de lancette (612) et/ou étant relié à un dispositif de serrage (610) pour serrer le système de lancette (612), de telle sorte que le système de lancette (612) est serré lorsque le module de magasin (110) est relié au module de diagnostic (112).

2. Système de diagnostic (114) selon la revendication précédente, **caractérisé par** un dispositif (126, 138, 418) d'échange de données pour échanger des informations sur le lot de rubans de tests (310) entre le module de magasin (110) et le module de diagnostic (112).

3. Système de diagnostic (114) selon la revendication précédente, **caractérisé en ce que** le dispositif (126, 138, 418) d'échange de données présente un transpondeur disposé dans le module de magasin (110) et un appareil (626) de lecture de transpondeur disposé dans le module de diagnostic (112).

4. Système de diagnostic (114) selon l'une des deux revendications précédentes, **caractérisé en ce que** le dispositif (126, 138, 418) d'échange de données présente une mémoire de données (418), en particulier une EPROM, disposée dans le module de magasin (110).

5. Système de diagnostic (114) selon l'une des trois revendications précédentes, **caractérisé en ce que** le dispositif (126, 138, 418) d'échange de données présente au moins un contact électrique (126, 138) disposé dans le dispositif de liaison (122, 130).

6. Système de diagnostic (114) selon l'une des revendications précédentes, **caractérisé en ce que** le dispositif de liaison (122, 130) présente un dispositif d'insertion (122, 130).

7. Système de diagnostic (114) selon la revendication précédente, **caractérisé en ce que** le dispositif d'insertion (122, 130) présente un guide à queue d'aronde (122, 130).

8. Système de diagnostic (114) selon l'une des revendications précédentes, **caractérisé en ce que** le dispositif de serrage (610) présente un coulisseau de serrage (616) relié à un système de ressort (614).

9. Système de diagnostic (114) selon l'une des revendications précédentes, **caractérisé en ce que** le module de diagnostic (112) présente un affichage (142) et/ou un système informatique (626).

10. Système de diagnostic (114) selon l'une des revendications précédentes, **caractérisé en ce que** le système de diagnostic (114) présente les éléments de commande (510, 148, 144) suivants : un dispositif d'actionnement (510) pour actionner le module de magasin (110), un dispositif de déclenchement (148) pour déclencher le système de lancette (612) et un système de commande (144) pour permettre de commander le module de diagnostic (112).

11. Système de diagnostic (114) selon la revendication précédente, **caractérisé par** une pièce essentiellement verticale de poignée (110) et une surface de commande (140) essentiellement horizontale.

12. Système de diagnostic (114) selon la revendication précédente, **caractérisé en ce que** la surface de commande (140) présente au moins un élément de commande (144).

13. Système de diagnostic (114) selon l'une des deux revendications précédentes, **caractérisé en ce qu'**au moins trois éléments de commande (510, 148, 144) peuvent être commandés d'une seule main lorsque le module de magasin (110) est relié au module de diagnostic (112).

14. Système de diagnostic (114) selon l'une des revendications précédentes, **caractérisé en ce que** le module de magasin (110) présente au moins une électrode de mesure (422), le module de diagnostic (112) pouvant être relié par la ou les électrodes de mesure (422) à au moins un ruban de test (310) lorsque le module de magasin (110) est relié au module de diagnostic (112).

15. Système de diagnostic (114) selon l'une des revendications précédentes, **caractérisé en ce que** le module de magasin (110) présente un magasin (412) pour piles de rubans de tests (310).

16. Système de diagnostic (114) selon l'une des revendications précédentes, **caractérisé en ce que** le module de magasin (110) présente un dispositif d'actionnement (510) pour extraire les rubans de tests (310).

17. Système de diagnostic (114) selon la revendication précédente, **caractérisé en ce que** le dispositif d'actionnement (510) présente les éléments suivants : un bouton d'actionnement (116), une tige de poussée (516) et une pièce de poussée (518) qui permet de déplacer un ruban de test (310), la pièce de poussée (518) présentant au moins une première arête de poussée (522) et une deuxième arête de poussée (524) qui saisissent un ruban de test (310).

18. Système de diagnostic (114) selon l'une des revendications précédentes, **caractérisé par** un moyen de séchage (416) installé dans le module de magasin (110) et permettant de diminuer l'humidité de l'air.

19. Système de diagnostic (114) selon l'une des revendications 1 à 18, dans lequel le dispositif de liaison (122, 130) présente un dispositif d'actionnement (510) pour actionner le module de magasin (110), en particulier pour délivrer au moins un ruban de test (310), et/ou est relié à un dispositif d'actionnement (510) pour actionner le module de magasin (110), en particulier pour délivrer au moins un ruban de test (310), de telle sorte qu'au moins un ruban de test (310) soit délivré lorsque le module de magasin (110) est relié au module de diagnostic (112).

20. Procédé d'utilisation d'un système de diagnostic (114) selon l'une des revendications précédentes, le procédé présentant les étapes suivantes :
a) un module de magasin (110) présentant des rubans de tests (310) est relié à un module de diagnostic (112), un système de lancette (612) est serré et/ou un ruban de test (310) est extrait,
b) le système de lancette (612) est déclenché, ce qui délivre une quantité d'échantillon liquide et en particulier une goutte de sang,
c) un ruban de test (310) extrait du module de magasin (110) est transporté dans une position de mesure,
d) la quantité d'échantillon est reliée au ruban de test (310) et
e) la concentration d'au moins une substance prédéterminée présente dans la quantité d'échantillon est déterminée.

21. Procédé selon la revendication précédente, **caractérisé en ce qu'**un système informatique (626) est démarré à l'étape de traitement a).

22. Procédé selon l'une des revendications précédentes, **caractérisé en ce qu'**à l'étape a) du procédé, des informations sur le lot des rubans de tests (310) sont échangées entre le module de magasin (110) et le module de diagnostic (112).

23. Procédé selon la revendication précédente, **caractérisé en ce que** lorsque l'étape e) du procédé est exécutée, les informations concernant le lot de rubans de tests (310) sont prises en compte.

24. Procédé selon l'une des revendications de procédé qui précèdent, **caractérisé par** l'étape supplémentaire suivante :
f) le ruban de test (310) est expulsé.

25. Procédé selon l'une des revendications de procédé qui précèdent, **caractérisé en ce qu'**un même dispositif d'actionnement (510) est actionné dans les étapes c) et f) du procédé.

26. Procédé selon l'une des revendications de procédé qui précèdent, **caractérisé en ce qu'**à l'étape e) du procédé, un courant est mesuré entre au moins deux électrodes reliées au ruban de test (310) en tant que fonction d'une tension.

27. Procédé selon l'une des revendications de procédé qui précèdent, **caractérisé en ce qu'**à l'étape b) du procédé, la quantité souhaitée d'échantillon liquide est ajustée sur le système de diagnostic (114) avant le déclenchement du système de lancette (612).
